# EUROPEAN PATENT APPLICATION

(11) **EP 4 026 821 A1**
(43) Date of publication of application: **13.07.2022**
(21) Application number: 20861184.8
(22) Date of filing: 04.09.2020
(51) Int. Cl.: C07B 37/04, C07C 41/30, C07C 43/225, C07C 201/12, C07C 205/06, C07D 333/10, C07D 333/38, C07F 5/02, C07D 209/42, C07D 307/06

(54) **MECHANOREDOX REACTION USING PIEZOELECTRIC MATERIAL, AND PRODUCTION METHOD USING SAID REACTION**

(30) Priority: 06.09.2019 JP 2019163323
(71) Applicant: National University Corporation Hokkaido University, Sapporo-shi, Hokkaido 060-0808 (JP)
(72) Inventor: ITO, Hajime, Sapporo-shi, Hokkaido 060-0808 (JP); KUBOTA, Koji, Sapporo-shi, Hokkaido 060-0808 (JP)
(74) Representative: Murgitroyd & Company
(86) International application number: PCT/JP2020/033650
(87) International publication number: WO 2021/045207

(57) **Abstract**

Disclosed are a method for producing a highly reactive intermediate, which comprises: preparing an electron-accepting active compound (1), preparing a piezoelectric material (3), and applying mechanical strain to the piezoelectric material (3) in the presence of the electron-accepting active compound (1) and the piezoelectric material (3), and subjecting the compound (1) to one-electron reduction to generate a corresponding highly reactive intermediate; a redox reaction method using the method for producing the same; and a method for producing a redox reaction product.

## Description

### [Technical Field]

The present invention relates to a mechanoredox reaction using a piezoelectric material, and more particularly to a mechanoredox reaction and a method for producing a redox reaction product using the reaction method.

### [Background Art]

In recent years, a photoredox reaction has been actively studied as an organic synthesis reaction utilizing electrical redox. In this reaction, a catalyst is excited by light irradiation, and electron transfer from the catalyst to a substrate or electron transfer from the substrate to the catalyst is utilized (see Non-Patent Literatures 1 to 4). However, this reaction requires a large amount of solvents and efficient stirring, which can lead to complicated reaction setup. Since light irradiation does not easily reach the inside of a reaction vessel, the efficiency is not always satisfactory. It is impossible to apply a reactant which does not transmit light.

### [Citation List]

### [Non-Patent Literature]

[NPL 1] Munetaka Akita and Takashi Koike, J. Synth. Org. Chem., Jpn, Vol. 74, No. 11, page 1036-1046, 2016.
[NPL 2] D. A. Nagib; D. W. MacMillan "Trifluoromethylation of arenes and heteroarenes by means of photoredox catalysis" Nature, 2011, 480, 224-228, 10.1038/nature10647.
[NPL 3] C. K. Prier; D. A. Rankic; D. W. MacMillan "Visible light photoredox catalysis with transition metal complexes: applications in organic synthesis" Chem Rev, 2013, 113, 5322-5363, 10.1021/cr300503r.
[NPL 4] D. M. Schultz; T. P. Yoon "Solar synthesis: prospects in visible light photocatalysis" Science, 2014, 343, 1239176, 10.1126/science.1239176.

### [Summary of Invention]

### [Technical Problem]

Although a photoredox reaction requires efficient light irradiation into the solution, light irradiation becomes increasingly difficult in a large reaction vessel and there is a need for strict removal of oxygen from the solution to avoid quenching of excited species (or intermediates) due to oxygen, which leads to a problem such as difficulty in scaling up and industrialization. In principle, there is a problem that the photoredox reaction cannot be used for the reaction of colored substances, suspended solutions and polymers which do not transmit light.

. Therefore, there is required a novel redox reaction which can reduce the amount of the solvent used and can be performed in the presence of the air, and also requires no light irradiation and is easier to scale up; and a novel method for producing a reaction product using the reaction. Such novel redox reaction is of great interest from academic and industrial points of view.

It is an object of the present disclosure to provide a novel method for generating (or producing) a highly reactive intermediate, a novel redox reaction method, and a method for producing a redox reaction product using such redox reaction.

### [Means for Solving the Problems]

As a result of intensive study, the present inventors have found that a redox reaction (also referred to as "mechanoredox reaction") can be allowed to proceed in the presence of a piezoelectric material by applying mechanical force using a ball mill to generate a highly reactive intermediate. They have also found that the redox reaction (or mechanoredox reaction) using the mechanical force can reduce an amount of a solvent and can be performed in the presence of air, and also requires no light irradiation and is easier to scale up, and thus the present invention has been completed.

The present disclosure includes the following embodiments.
1. A method for generating (or producing) a highly reactive intermediate, which comprises:
   preparing an electron-accepting active compound (1);
   preparing a piezoelectric material (3); and
   applying mechanical strain (force) to the piezoelectric material (3) in the presence of the electron-accepting active compound (1) and the piezoelectric material (3), and subjecting the compound (1) to one-electron reduction to generate a corresponding highly reactive intermediate.
2. The method for generating (or producing) a highly reactive intermediate according to the above-mentioned 1, wherein the electron-accepting active compound (1) is selected from
   an aryl compound having a leaving group represented by the following general formula (I-1):

      A¹ -Xn

      wherein A¹ is selected from an optionally substituted aryl group and an optionally substituted heteroaryl group, X is a leaving group, and n is an integer of 1 or more,
   a trifluoromethyl compound selected from an optionally substituted trifluoromethyl-dibenzothiophene (I-2a), an optionally substituted trifluoromethyl-diphenylmercaptan (I-2b) and trifluoromethanesulfonyl chloride (I-2c) represented by the following formulas (I-2a) to (I-2c): 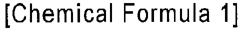 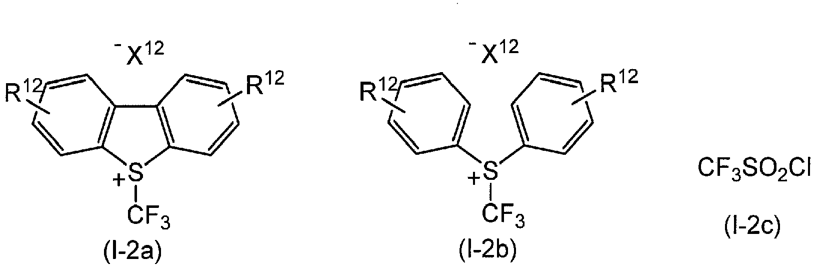 wherein R^{1 2} each independently comprises hydrogen, an alkyl group, an alkoxy group, a cycloalkyl group, an alkenyl group, an alkynyl group, an aryl group, an aryloxy group, an acyl group, an alkoxycarbonyl group, an amino group, fluorine, chlorine, a cyano group, a nitro group, etc., R^{1 2} (s) may be crosslinked to each other to form a cyclic structure, and also may have other substituents, R^{1 2} may be interrupted, for example, with an oxygen atom, a sulfur atom, a nitrogen atom, a carbonyl group, an ester bond, etc., and ⁻ X^{1 2} represents an anion, and
   a bromide or iodide represented by the following general formula (I-3):
      CBr₄ (I-3a) or CHI₃ (I-3b), and
   an activated fatty acid represented by the following general formula (I-4): 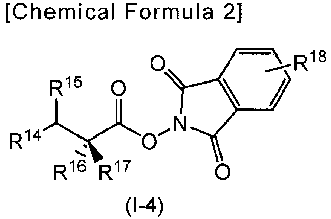 wherein R^{1 4} to R^{1 7} may be the same or different from each other, and are each independently selected from hydrogen, an optionally substituted alkyl group, an optionally substituted alkenyl group, an optionally substituted alkynyl group, an optionally substituted alkoxy group, an optionally substituted aryl group and an optionally substituted heteroaryl group; R^{1 4} and R^{1 5}, R^{1 4} and R^{1 6}, R^{1 5} and R^{1 7}, R^{1 6} and R^{1 7}, etc. may be bonded to each other to form a ring, and R^{1 8} may be hydrogen or a substituent.
3. The generation (or production) method according to the above-mentioned item 2, wherein the electron-accepting active compound (1) is selected from an aryl compound having a leaving group represented by the formula (I-1):
   wherein, in the general formula (1-1), the optionally substituted aryl group as for A¹ comprises a phenyl group, a naphthyl group, an anthracenyl group, a phenanthrenyl group, a biphenyl group, a terphenyl group, a pyrenyl group, a perylenyl group and a triphenyleny group,
   the optionally substituted heteroaryl group as for A¹ comprises a sulfur-containing heteroaryl group, an oxygen-containing heteroaryl group, a nitrogen-containing heteroaryl group, and a heteroaryl group containing two or more heteroatoms, and
   the leaving group X comprises iodine, bromine, chlorine and a diazonium salt.
4. The generation (or production) method according to any one of the above-mentioned items 1 to 3, wherein the piezoelectric material (3) includes at least one selected from barium titanate, strontium titanate, lithium niobate, tourmaline, quartz, topaz, sucrose, Rochelle salt (KNaC₄ H₄ O₆ ·4H₂ O), gallium orthophosphate (GaPO₄ ), langasite (La₃ Ga₅ SiO_{1 4} ), lead titanate (PbTiO₃ ), lead zirconate titanate, potassium niobate (KNbO₃ ), lithium tantalate (LiTaO₃ ), sodium tungstate (NaXWO₃ ), zinc oxide (ZnO, Zn₂ O₃ ), Ba₂ NaNb₅ O₅, Pb₂ KNb₅ O_{1 5}, lithium tetrabolate (Li₂ B₄ O₇ ), sodium potassium niobate ((K,Na)NbO₃ ), bismuth ferrite (BiFeO₃ ), sodium niobate (NaNbO₃ ), bismuth titanate (Bi₄ Ti₃ O_{1 2} ), sodium bismuth titanate (Na_{0 . 5} B_{i 0 . 5} TiO₃ ), polyvinylidene fluoride, aluminum nitride (AIN), gallium phosphate (GaPO₄ ) and gallium arsenic (GaAs).
5. The generation (or production) method according to any one of the above-mentioned items 1 to 4, wherein the piezoelectric material (3) is allowed to be present in an amount of 0.5 mol% or more and 1,500 mol% or less on the basis(100%) of the number of mols obtained by multiplying the number of mols of the compound (1) by a valence.
6. The generation (or production) method according to any one of the above-mentioned items 1 to 5, wherein the highly reactive intermediate includes at least one selected from a radical, an anion radical and an anion.
7. A redox reaction method comprising the method for generating (or producing) a highly reactive intermediate according to any one of the above-mentioned items 1 to 6, the redox reaction method comprising:
   subjecting the highly reactive intermediate to a redox reaction to produce a redox reaction product.
8. The redox reaction method according to the above-mentioned item 7, which comprises:
   preparing an electron-accepting active compound (1);
   preparing a piezoelectric material (3); and
   applying mechanical strain to the piezoelectric material (3) in the presence of the electron-accepting active compound (1) and the piezoelectric material (3), and subjecting the compound (1) to one-electron reduction to generate a corresponding highly reactive intermediate, followed by a redox reaction of the highly reactive intermediate by itself to produce a redox reaction product.
9. The redox reaction method according to the above-mentioned 8, wherein the redox reaction of the electron-accepting active compound (1) by itself comprises that the activated fatty acid represented by the formula (I-4) as the electron-accepting active compound (1) undergoes a redox reaction by itself.
10. The redox reaction method according to the above-mentioned item 7, which comprises:
   further preparing at least one compound (2) selected from an aromatic compound (2-1) optionally containing a heteroatom, a diboronic acid ester (2-2) and an aliphatic alcohol (2-3); and
   applying mechanical strain to the piezoelectric material (3) in the presence of the compound (2), in addition to the electron-accepting active compound (1) and the piezoelectric material (3), and subjecting the compound (1) to one-electron reduction to generate a corresponding highly reactive intermediate, followed by a reaction of the highly reactive intermediate with the compound (2) to produce a redox reaction product.
11. The redox reaction method according to the above-mentioned item 10, wherein the redox reaction between the electron-accepting active compound (1) and the compound (2) is selected from:
   a redox reaction between an aryl compound having a leaving group represented by the formula (1-1) as the electron-accepting active compound (1), and an aromatic compound (2-1) optionally containing a heteroatom or a diboronic acid ester (2-2) as the compound (2);
   a redox reaction between trifluoromethyl compounds represented by the formulas (I-2a) to (I-2c) as the electron-accepting active compound (1), and an aromatic compound (2-1) optionally containing a heteroatom as the compound (2); and
   a redox reaction between a bromide or iodide represented by the formula (I-3) as the electron-accepting active compound (1) and an aliphatic alcohol (2-3) as the compound (2).
12. The redox reaction method according to any one of the above-mentioned items 7 and 10 to 11, wherein the compound (2) is selected from:
   an aromatic compound (2-1) optionally containing a heteroatom represented by the following general formula (II-1):

      A² -H

      wherein A² is selected from an optionally substituted aryl group and an optionally substituted heteroaryl group;
   a diboronic acid ester (2-2) represented by the following general formula (II-2): 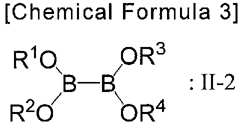 wherein R¹ to R⁴ are each independently selected from hydrogen, an optionally substituted alkyl group and an optionally substituted aryl group, R¹ and R² may be bonded to each other, and R³ and R⁴ may be bonded to each other; and
   an aliphatic alcohol (2-3) represented by the following general formula (II-3): 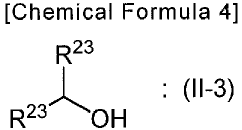 wherein R^{2 3} may be the same or different from each other, and are each independently selected from hydrogen, an optionally substituted alkyl group, an optionally substituted alkenyl group, an optionally substituted alkynyl group and an optionally substituted aryl group, and R^{2 3} and R^{2 3} may be bonded to each other.
13. The redox reaction method according to any one of the above-mentioned items 7 and 10 to 12, wherein the aromatic group in the aromatic compound (2-1) optionally containing a heteroatom can be selected from an optionally substituted aryl group and an optionally substituted heteroaryl group,
   the optionally substituted aryl group comprises a phenyl group, a naphthyl group, an anthracenyl group, a phenanthrenyl group, a biphenyl group, a terphenyl group, a pyrenyl group, a perylenyl group, a triphenyleny group and a coronenyl group,
   the optionally substituted heteroaryl group comprises a sulfur-containing heteroaryl group, an oxygen-containing heteroaryl group, a nitrogen-containing heteroaryl group, and a heteroaryl group containing two or more heteroatoms,
   the diboronic acid ester (2-2) comprises a diboronic acid alkyl ester, a diboronic acid alkylene glycol ester, a diboronic acid aryl ester, a diboronic acid arylene glycol ester and tetrahydroxydiboran, and
   the aliphatic alcohol (2-3) comprises a primary aliphatic alcohol and a secondary aliphatic alcohol.
14. The redox reaction method according to any one of the above-mentioned items 7 and 10 to 13, wherein the equivalent ratio of the compound (1) to the compound (2) (compound (1)/compound (2)) is 10/1 to 1/10.
15. A method for producing a redox reaction product, which comprises using the redox reaction method according to any one of the above-mentioned items 7 to 14.

### [Advantageous Effects of Invention]

The (mechano)redox reaction method and the method for generating (or producing) a reaction product according to an embodiment of the present invention can reduce an amount of a solvent and can be performed in the presence of air, and also requires no light irradiation and is easier to scale up.

### [Description of Embodiments]

The present invention provides, in one aspect, a method for generating (or producing) a highly reactive intermediate, which comprises:
preparing an electron-accepting active compound (1);
preparing a piezoelectric material (3); and
applying mechanical strain (force) to the piezoelectric material (3) in the presence of the electron-accepting active compound (1) and the piezoelectric material (3), and subjecting the compound (1) to one-electron reduction to generate a corresponding highly reactive intermediate.

The method for generating (or producing) a highly reactive intermediate according to an embodiment of the present invention comprises preparing an electron-accepting active compound (1).

In the embodiment of the present invention, the electron-accepting active compound (1) is a compound which receives electrons generated from the piezoelectric material (3) to which physical stress (or strain) is applied, to produce a highly reactive intermediate. The electron-accepting active compound (1) is not particularly limited as long as it can generate a highly reactive intermediate (e.g., a radical, an anion radical, an anion, etc.) and is preferably a compound capable of undergoing a redox reaction.

The electron-accepting active compound (1) is preferably selected from an aryl compound having a leaving group represented by the following general formula (I-1):

A¹ -Xn

[wherein A¹ is selected from an optionally substituted aryl group and an optionally substituted heteroaryl group, X is a leaving group, and n is an integer of 1 or more],
a trifluoromethyl compound selected from an optionally substituted trifluoromethyl-dibenzothiophene (I-2a), an optionally substituted trifluoromethyldiphenylmercaptan (I-2b) and trifluoromethanesulfonyl chloride (I-2c) represented by the following formulas (I-2a) to (I-2c): 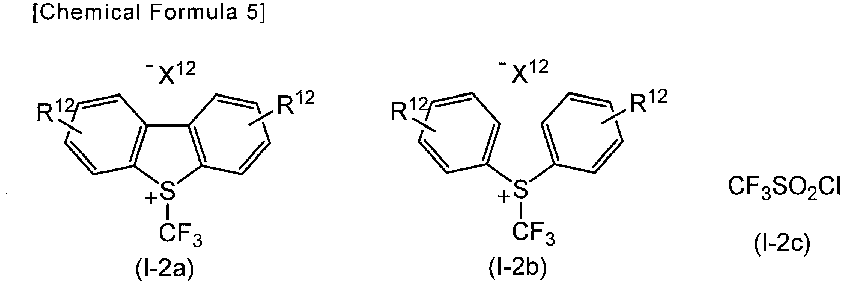 [wherein R^{1 2} each independently include hydrogen, an alkyl group, an alkoxy group, a cycloalkyl group, an alkenyl group, an alkynyl group, an aryl group, an aryloxy group, an acyl group, an alkoxycarbonyl group, an amino group, fluorine, chlorine, a cyano group, a nitro group, etc., R^{1 2} (s) may be crosslinked to each other to form a cyclic structure, and also may have other substituents, R^{1 2} may be interrupted, for example, with an oxygen atom, a sulfur atom, a nitrogen atom, a carbonyl group, an ester bond, etc., and ⁻ X^{1 2} represents an anion],
a bromide or iodide represented by the following general formula (I-3):
   CBr₄ (I-3a) or CHI₃ (I-3b), and
an activated fatty acid represented by the following general formula (I-4): 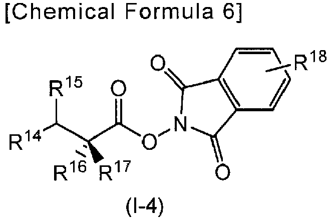 [wherein R^{1 4} to R^{1 7} may be the same or different from each other, and are each independently selected from hydrogen, an optionally substituted alkyl group, an optionally substituted alkenyl group, an optionally substituted alkynyl group, an optionally substituted alkoxy group, an optionally substituted aryl group and an optionally substituted heteroaryl group; R^{1 4} and R^{1 5}, R^{1 4} and R^{1 6}, R^{1 5} and R^{1 7}, R^{1 6} and R^{1 7}, etc. may be bonded to each other to form a ring, and R^{1 8} may be hydrogen or a substituent].

The above-mentioned aryl compound having a leaving group represented by the formula (1-1) is not particularly limited as long as it is a compound which can receive electrons generated from the piezoelectric material (3) to which physical stress (or strain) is applied, to generate a highly reactive intermediate (e.g., an optionally substituted aryl radical or an optionally substituted heteroaryl radical).

In the general formula (1-1),
the leaving group X can include, for example, iodine, bromine, chlorine and a diazonium salt, and preferably includes a diazonium salt.

In the general formula (1-1),
the optionally substituted aryl group (or an aromatic hydrocarbon group) as for A¹ can include, for example, a phenyl group, a naphthyl group, an anthracenyl group (or an anthracene group), a phenanthrenyl group (or a phenanthrene group), a biphenyl group, a terphenyl group, a pyrenyl group (or a pyrene group), a perylenyl group (or a perylene group) and a triphenylenyl group (or a triphenylene group).

It is preferable to include a phenyl group, a naphthyl group, an anthracenyl group, a phenanthrenyl group, a biphenyl group and a terphenyl group.

In the general formula (I-1),
the optionally substituted heteroaryl group (or a heteroaromatic group) as for A¹ can include, for example:
sulfur-containing heteroaryl groups such as a thiophenyl group (a thiophene group or a thienyl group), a benzothienyl group and a dibenzothienyl group;
oxygen-containing heteroaryl groups such as a furanyl group (or a furan group), a benzofuranyl group, a dibenzofuranyl group, a phenyldibenzofuranyl group and a dibenzofuranylphenyl group;
nitrogen-containing heteroaryl groups such as a pyronyl group (or a pyrrol group), a benzopyronyl group, a dibenzopyronyl group, a pyridyl group (or a pyridine group), a pyrimidinyl group (or a pyrimidine group), a pyrazyl group (or a pyrazine group), a quinolyl group (or a quinoline group), an isoquinolyl group (or an isoquinoline group), a carbazolyl group (or a carbazole group), a 9-phenylcarbazolyl group, an acridinyl group (or an acridine group), a quinazolyl group (or a quinazoline group), a quinoxalyl group (or a quinoxaline group), a 1,6-naphthyldinyl group, a 1,8-naphthyldinyl group and a porphyrin group (or a porphyrin ring); and
heteroaryl groups containing two or more heteroatoms (e.g., nitrogen and sulfur), such as a benzothiazolyl group (or a benzothiazole group).

It is preferable to include a thiophenyl group, a furyl group, a pyronyl group, a benzthiophenyl group, a benzfuranyl group, a benzpyronyl group, a dibenzthiophenyl group, a dibenzfuranyl group and a dibenzpyronyl group.

The substituent, with which the aryl group and the heteroaryl group can be substituted, is not particularly limited as long as the objective redox reaction of the present invention can be performed.

The substituent includes, for example:
alkyl groups having 1 to 24 carbon atoms, for example, 1 to 18 carbon atoms, for example, 1 to 12 carbon atoms, for example, 1 to 8 carbon atoms (e.g., a methyl group, an ethyl group, an n-propyl group, an isopropyl group, an n-butyl group, an isobutyl group, a tert-butyl group, a pentyl group, a hexyl group, an octyl group, etc.),
alkoxy groups having 1 to 24 carbon atoms, for example, 1 to 18 carbon atoms, for example, 1 to 12 carbon atoms, for example, 1 to 8 carbon atoms (e.g., a methoxy group, an ethoxy group, an n-propoxy group, an isopropoxy group, an n-butoxy group, an isobutoxy group, a tert-butoxy group, a pentyloxy group, a hexyloxy group, an octyloxy group, etc.),
cycloalkyl groups having 3 to 24 carbon atoms, for example, 3 to 18 carbon atoms, for example, 3 to 12 carbon atoms, for example, 3 to 8 carbon atoms (e.g., a cyclopropyloxy group, a cyclobutyloxy group, a cyclopentyloxy group, a cyclohexyloxy group, etc.),
alkenyl groups having 1 to 24 carbon atoms, for example, 1 to 18 carbon atoms, for example, 1 to 12 carbon atoms, for example, 1 to 8 carbon atoms (e.g., an ethenyl group, a propenyl group, a butenyl group, a pentenyl group, a hexenyl group, an octenyl group, etc.),
alkynyl groups having 1 to 24 carbon atoms, for example, 1 to 18 carbon atoms, for example, 1 to 12 carbon atoms, for example, 1 to 8 carbon atoms (e.g., an ethynyl group, a propynyl group, a butynyl group, a pentynyl group, a hexynyl group, an octynyl group, etc.),
aryl groups having 5 to 24 carbon atoms, for example, 5 to 18 carbon atoms, for example, 5 to 12 carbon atoms, for example, 5 to 8 carbon atoms (e.g., a phenyl group, a naphthyl group, a biphenyl group, etc.),
aryloxy groups having 5 to 24 carbon atoms, for example, 5 to 18 carbon atoms, for example, 5 to 12 carbon atoms, for example, 5 to 8 carbon atoms (e.g., a phenoxy group, a naphthyloxy group, a biphenyloxy group, etc.),
heteroaryl groups having 4 to 24 carbon atoms, for example, 1 to 18 carbon atoms, for example, 1 to 12 carbon atoms, for example, 1 to 8 carbon atoms (e.g., a thiophenyl group, a furanyl group, a carbazole group, a benzothiophenyl group, a benzofuranyl group, an indolyl group, a pyrrolyl group, a pyridyl group, etc.),
acyl groups having 1 to 24 carbon atoms, for example, 1 to 18 carbon atoms, for example, 1 to 12 carbon atoms, for example, 1 to 8 carbon atoms (e.g., an acetyl group, a propionyl group, a butanoyl group, a pentanoyl group, a heptanoyl group, a group in which a carbonyl group included in the acyl group is substituted with an ester group, an amide group, etc.),
alkoxycarbonyl groups having 1 to 24 carbon atoms, for example, 1 to 18 carbon atoms, for example, 1 to 12 carbon atoms, for example, 1 to 8 carbon atoms (e.g., a methoxycarbonyl group, an ethoxycarbonyl group, a propoxycarbonyl group, a butoxycarbonyl group, a tert-butoxycarbonyl group, a pentoxycarbonyl group, etc.),
amino groups having 1 to 24 carbon atoms, for example, 1 to 18 carbon atoms, for example, 1 to 12 carbon atoms, for example, 1 to 8 carbon atoms (e.g., a diphenylamino group, a dimethylamino group, etc.), and
fluorine (including partial fluorine substitution and complete fluorine substitution), a cyano group and a nitro group.
The substituents may be crosslinked to each other, and the entire substituent may form a cyclic structure (an aromatic group). Further, the above-mentioned substituent may be further substituted with the above-mentioned substituent.

More specifically, it is possible to exemplify, as the aryl compound having a leaving group represented by the general formula (1-1) as for A¹ , for example, the following groups:
naphthyl groups such as a naphthyl group and an aryl (e.g., phenyl, etc.) naphthyl group;
phenanthrenyl groups;
anthracenyl groups such as an anthracenyl group, an aryl (e.g., phenyl, etc.) anthracenyl group and a diaryl (e.g., dinaphthyl, etc.) anthracenyl group;
pyrenyl groups such as a pyrenyl group and an alkyl (e.g., t-butyl, etc.) pyrenyl group;
biphenyl groups such as a biphenyl group and an biphenyl group having an alkylene (e.g., propylene, isopropylene, etc.) crosslink;
phenyl groups such as a phenyl group, an alkyl (e.g., methyl, t-butyl) phenyl group, a dialkyl (e.g., dimethyl) phenyl group, an alkoxy (e.g., methoxy) phenyl group, a dialkylamino (e.g., dimethylamino) phenyl group, a diaryl (e.g., diphenyl) aminophenyl group, a perfluoroalkyl (e.g., trifluoromethyl) phenyl group, an alkyl (e.g., ethyl) oxycarbonylphenyl group, an alkanoyl (e.g., acyl) phenyl group, a fluorophenyl group, a chlorophenyl group, a bromophenyl group, a nitrophenyl group and a cyanophenyl group;
aryl (e.g., phenyl, etc.) substituted carbazolyl groups;
anthracene-9.10-dione groups; and
aryl (e.g., phenyl, etc.) substituted thienyl groups and alkyloxycarbonyl (e.g., methyloxycarbonyl, etc.) substituted thienyl groups.

It is possible to use, as the aryl compound having a leaving group (an aromatic compound), commercially available compounds.

In the embodiment of the present invention,
the trifluoromethyl compound selected from an optionally substituted trifluoromethyl-dibenzothiophene (I-2a), an optionally substituted trifluoromethyldiphenylmercaptan (I-2b) and trifluoromethanesulfonyl chloride (I-2c) represented by the following formulas (I-2a) to (I-2c): 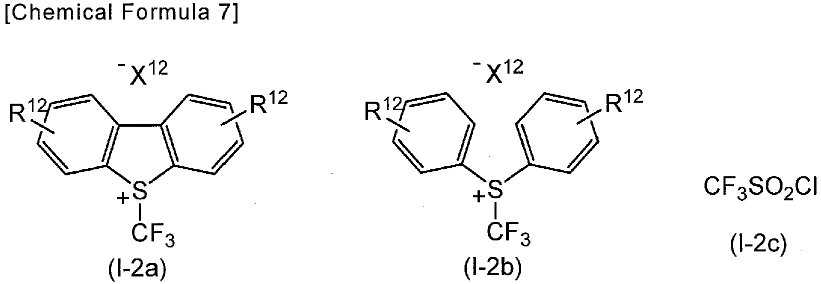 [wherein R^{1 2} each independently include hydrogen, an alkyl group, an alkoxy group, a cycloalkyl group, an alkenyl group, an alkynyl group, an aryl group, an aryloxy group, an acyl group, an alkoxycarbonyl group, an amino group, fluorine, chlorine, a cyano group, a nitro group, etc., R^{1 2} (s) may be crosslinked to each other to form a cyclic structure, and also may have other substituents, R^{1 2} may be interrupted, for example, with an oxygen atom, a sulfur atom, a nitrogen atom, a carbonyl group, an ester bond, etc., and ⁻ X^{1 2} represents an anion], is not particularly limited as long as it is a compound which can receive electrons generated from the piezoelectric material (3) to which physical stress (or strain) is applied, to generate a trifluoromethyl radical.

For each of the formulas (I-2a) and (I-2b), R^{1 2} may or may not be present, R^{1 2} may or may not be the same, and the number of R^{1 2} (s) to be present is particularly limited and may be 2 or more. In the case of the formula (I-2a), the number of R^{1 2} (s) to be present may be 2 to 8, or 2 to 6 In the case of the formula (I-2b), the number of R^{1 2} (s) to be present may be 2 to 10, 2 to 8, or 2 to 6.

R^{1 2} is not particularly limited as long as it receive electrons generated from the piezoelectric material (3) and each of the formulas (I-2a) and (I-2b) can generate a trifluoromethyl radical.

R^{1 2} can include, for example, hydrogen, an alkyl group, an alkoxy group, a cycloalkyl group, an alkenyl group, an alkynyl group, an aryl group, an aryloxy group, an acyl group, an alkoxycarbonyl group, an amino group, fluorine, chlorine, a cyano group, and a nitro group. Two or more R^{1 2} (s) may be present, R^{1 2} (s) may be crosslinked to each other, R^{1 2} may form a cyclic structure, and R^{1 2} may have other substituents. R^{1 2} may be interrupted, for example, with an oxygen atom, a sulfur atom, a nitrogen atom, a carbonyl group, an ester bond, etc.

R^{1 2} includes, for example:
alkyl groups having 1 to 24 carbon atoms, for example, 1 to 18 carbon atoms, for example, 1 to 12 carbon atoms, for example, 1 to 8 carbon atoms (e.g., a methyl group, an ethyl group, an n-propyl group, an isopropyl group, an n-butyl group, an isobutyl group, a tert-butyl group, a pentyl group, a hexyl group, an octyl group, etc.),
alkoxy groups having 1 to 24 carbon atoms, for example, 1 to 18 carbon atoms, for example, 1 to 12 carbon atoms, for example, 1 to 8 carbon atoms (e.g., a methoxy group, an ethoxy group, an n-propoxy group, an isopropoxy group, an n-butoxy group, an isobutoxy group, a tert-butoxy group, a pentyloxy group, a hexyloxy group, an octyloxy group, etc.),
cycloalkyl groups having 3 to 24 carbon atoms, for example, 3 to 18 carbon atoms, for example, 3 to 12 carbon atoms, for example, 3 to 8 carbon atoms (e.g., a cyclopropyloxy group, a cyclobutyloxy group, a cyclopentyloxy group, a cyclohexyloxy group, etc.),
alkenyl groups having 1 to 24 carbon atoms, for example, 1 to 18 carbon atoms, for example, 1 to 12 carbon atoms, for example, 1 to 8 carbon atoms (e.g., an ethenyl group, a propenyl group, a butenyl group, a pentenyl group, a hexenyl group, an octenyl group, etc.),
alkynyl groups having 1 to 24 carbon atoms, for example, 1 to 18 carbon atoms, for example, 1 to 12 carbon atoms, for example, 1 to 8 carbon atoms (e.g., an ethynyl group, a propynyl group, a butynyl group, a pentynyl group, a hexynyl group, an octynyl group, etc.),
aryl groups having 5 to 24 carbon atoms, for example, 5 to 18 carbon atoms, for example, 5 to 12 carbon atoms, for example, 5 to 8 carbon atoms (e.g., a phenyl group, a naphthyl group, a biphenyl group, etc.),
aryloxy groups having 5 to 24 carbon atoms, for example, 5 to 18 carbon atoms, for example, 5 to 12 carbon atoms, for example, 5 to 8 carbon atoms (e.g., a phenoxy group, a naphthyloxy group, a biphenyloxy group, etc.),
heteroaryl groups having 4 to 24 carbon atoms, for example, 1 to 18 carbon atoms, for example, 1 to 12 carbon atoms, for example, 1 to 8 carbon atoms (e.g., a thiophenyl group, a furanyl group, a carbazole group, a benzothiophenyl group, a benzofuranyl group, an indolyl group, a pyrrolyl group, a pyridyl group, etc.),
acyl groups having 1 to 24 carbon atoms, for example, 1 to 18 carbon atoms, for example, 1 to 12 carbon atoms, for example, 1 to 8 carbon atoms (e.g., an acetyl group, a propionyl group, a butanoyl group, a pentanoyl group, a heptanoyl group, a group in which a carbonyl group included in the acyl group is substituted with an ester group or an amide group, etc.),
alkoxycarbonyl groups having 1 to 24 carbon atoms, for example, 1 to 18 carbon atoms, for example, 1 to 12 carbon atoms, for example, 1 to 8 carbon atoms (e.g., a methoxycarbonyl group, an ethoxycarbonyl group, a propoxycarbonyl group, a butoxycarbonyl group, a tert-butoxycarbonyl group, a pentoxycarbonyl group, etc.),
amino groups having 1 to 24 carbon atoms, for example, 1 to 18 carbon atoms, for example, 1 to 12 carbon atoms, for example, 1 to 8 carbon atoms (e.g., a diphenylamino group, a dimethylamino group, etc.), and
fluorine (including partial fluorine substitution and complete fluorine substitution), chlorine (including partial chlorine substitution and complete chlorine substitution), a cyano group and a nitro group.

R^{1 2} (s) may be crosslinked to each other, and entire R^{1 2} may form a cyclic structure (an aromatic group). R^{1 2} may have the above substituents exemplified as for R^{1 2}. R^{1 2} may be interrupted, for example, with an oxygen atom, a sulfur atom, a nitrogen atom, a carbonyl group, an ester bond, etc.

For each of the formulas (I-2a) and (I-2b), ⁻ X^{1 2} is not particularly limited as long as each of the formulas (I-2a) and (I-2b) receives electrons generated from the piezoelectric material (3) to generate a trifluoromethyl radical.

⁻ X^{1 2} represents an anion and can include, for example, a trifluoromethanesulfonate group, a tetrafluoroborate group, etc.

In the embodiment of the present invention,
it is possible to use, as the electron-accepting active compound (1), a bromide or iodide represented by the following general formula (I-3):
CBr₄ (I-3a) or CHI₃ (I-3b)

Use of CBr₄ (I-3a) enables bromination, and use of CHI₃ (I-3b) enables iodination.

In the embodiment of the present invention,
it is possible to use, as the electron-accepting active compound (1), an activated fatty acid represented by the following general formula (I-4): 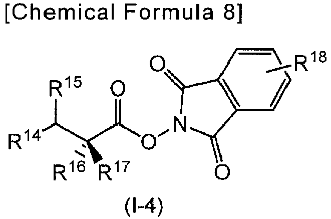 [wherein R^{1 4} to R^{1 7} may be the same or different from each other, and are each independently selected from hydrogen, an optionally substituted alkyl group, an optionally substituted alkenyl group, an optionally substituted alkynyl group, an optionally substituted alkoxy group, an optionally substituted aryl group and an optionally substituted heteroaryl group, R^{1 4} and R^{1 5}, R^{1 4} and R^{1 6}, R^{1 5} and R^{1 7}, R^{1 6} and R^{1 7}, etc. may be bonded to each other to form a ring, and R^{1 8} may be hydrogen or a substituent]. The activated fatty acid is not particularly limited as long as it can receive electrons generated from the piezoelectric material (3) to produce a highly reactive intermediate, and it is preferable that it can desorb carbon dioxide and undergoes a redox reaction by itself (without reacting with other compounds), to produce an olefin.

For the activated fatty acid represented by the formula (I-4), R^{1 4} to R^{1 7} may be the same or different from each other.

R^{1 4} to R^{1 7} are not particularly limited as long as the compound of the formula (I-4) can receive electrons generated from the piezoelectric material (3) to generate a highly reactive intermediate.

R^{1 4} to R^{1 7} can be selected, for example, from hydrogen, an optionally substituted alkyl group, an optionally substituted alkenyl group, an optionally substituted alkynyl group, an optionally substituted alkoxy group, an optionally substituted aryl group and an optionally substituted heteroaryl group.

The optionally substituted alkyl group includes, for example, a methyl group, an ethyl group, a propyl group, an isopropyl group, a butyl group, an isobutyl group, a t-butyl group, a pentyl group, a hexyl group, an octyl group, etc.

The optionally substituted alkenyl group includes, for example, an ethenyl group, a propenyl group, an isopropenyl group, a butenyl group, an isobutenyl group, a pentenyl group, etc.

The optionally substituted alkynyl group includes, for example, an acetylenyl group, a propynyl group, a butynyl group, a pentynyl group, a hexynyl group, etc.

The optionally substituted alkoxy group includes, for example, a methoxy group, an ethoxy group, a propoxy group, an isopropoxy group, a butoxy group, an isobutoxy group, a t-butoxy group, a pentoxy group, a hexoxy group, an octoxy group, etc.

The optionally substituted aryl group includes, for example, a phenyl group, a naphthyl group, a biphenyl group, etc.

The optionally substituted heteroaryl group includes, for example, a thiophenyl group, a furanyl group, a pyrrol group, a carbazole group, etc.

The above-mentioned substituent, which may be included, may be selected from an alkyl group, an aryl group, an alkoxy group, an aryloxy group, an ester group, a carbonyl group, an amino group, a cyano group, a nitro group, fluorine, chlorine, etc. R^{1 4} to R^{1 7} may be crosslinked to each other and may form a ring. The substituent may be further substituted. R^{1 4} to R^{1 7} and the substituent, which may be included, may be interrupted with oxygen, nitrogen, a sulfur atom, a carbonyl group, an ester group, etc.

For the formula (I-4), R^{1 8} is hydrogen or a substituent, and the number of R^{1 8} (s) is not particularly limited.

R^{1 8} is not particularly limited as long as it receives electrons generated from the piezoelectric material (3) and the formula (I-4) can generate a highly reactive intermediate.

R^{1 8} can include, for example, hydrogen, an alkyl group, an alkoxy group, a cycloalkyl group, an alkenyl group, an alkynyl group, an aryl group, an aryloxy group, an acyl group, an alkoxycarbonyl group, an amino group, fluorine, chlorine, a cyano group, a nitro group, etc. , The number of R^{1 8} (s) to be present may be 2 or more, R^{1 8} may be crosslinked to each other. R^{1 8} may form a cyclic structure, and R^{1 8} may have other substituents. R^{1 8} may be interrupted, for example, with an oxygen atom, a sulfur atom, a nitrogen atom, a carbonyl group, an ester bond, etc.

R^{1 8} includes, for example:
alkyl groups having 1 to 24 carbon atoms, for example, 1 to 18 carbon atoms, for example, 1 to 12 carbon atoms, for example, 1 to 8 carbon atoms (e.g., a methyl group, an ethyl group, an n-propyl group, an isopropyl group, an n-butyl group, an isobutyl group, a tert-butyl group, a pentyl group, a hexyl group, an octyl group, etc.),
alkoxy groups having 1 to 24 carbon atoms, for example, 1 to 18 carbon atoms, for example, 1 to 12 carbon atoms, for example, 1 to 8 carbon atoms (e.g., a methoxy group, an ethoxy group, an n-propoxy group, an isopropoxy group, an n-butoxy group, an isobutoxy group, a tert-butoxy group, a pentyloxy group, a hexyloxy group, an octyloxy group, etc.),
cycloalkyl groups having 3 to 24 carbon atoms, for example, 3 to 18 carbon atoms, for example, 3 to 12 carbon atoms, for example, 3 to 8 carbon atoms (e.g., a cyclopropyloxy group, a cyclobutyloxy group, a cyclopentyloxy group, a cyclohexyloxy group, etc.),
alkenyl groups having 1 to 24 carbon atoms, for example, 1 to 18 carbon atoms, for example, 1 to 12 carbon atoms, for example, 1 to 8 carbon atoms (e.g., an ethenyl group, a propenyl group, a butenyl group, a pentenyl group, a hexenyl group, an octenyl group, etc.),
alkynyl groups having 1 to 24 carbon atoms, for example, 1 to 18 carbon atoms, for example, 1 to 12 carbon atoms, for example, 1 to 8 carbon atoms (e.g., an ethynyl group, a propynyl group, a butynyl group, a pentynyl group, a hexynyl group, an octynyl group, etc.),
aryl groups having 5 to 24 carbon atoms, for example, 5 to 18 carbon atoms, for example, 5 to 12 carbon atoms, for example, 5 to 8 carbon atoms (e.g., a phenyl group, a naphthyl group, a biphenyl group, etc.),
aryloxy groups having 5 to 24 carbon atoms, for example, 5 to 18 carbon atoms, for example, 5 to 12 carbon atoms, for example, 5 to 8 carbon atoms (e.g., a phenoxy group, a naphthyloxy group, a biphenyloxy group, etc.),
heteroaryl groups having 4 to 24 carbon atoms, for example, 1 to 18 carbon atoms, for example, 1 to 12 carbon atoms, for example, 1 to 8 carbon atoms (e.g., a thiophenyl group, a furanyl group, a carbazole group, a benzothiophenyl group, a benzofuranyl group, an indolyl group, a pyrrolyl group, a pyridyl group, etc.),
acyl groups having 1 to 24 carbon atoms, for example, 1 to 18 carbon atoms, for example, 1 to 12 carbon atoms, for example, 1 to 8 carbon atoms (e.g., an acetyl group, a propionyl group, a butanoyl group, a pentanoyl group, a heptanoyl group, a group in which a carbonyl group included in the acyl group is substituted with an ester group or an amide group, etc.),
alkoxycarbonyl groups having 1 to 24 carbon atoms, for example, 1 to 18 carbon atoms, for example, 1 to 12 carbon atoms, for example, 1 to 8 carbon atoms (e.g., a methoxycarbonyl group, an ethoxycarbonylcarbonyl group, a propoxycarbonyl group, a butoxycarbonyl group, a tert-butoxycarbonyl group, a pentoxycarbonyl group, etc.),
amino groups having 1 to 24 carbon atoms, for example, 1 to 18 carbon atoms, for example, 1 to 12 carbon atoms, for example, 1 to 8 carbon atoms (e.g., a diphenylamino group, a dimethylamino group, etc.), and
fluorine (including partial fluorine substitution and complete fluorine substitution), chlorine (including partial chlorine substitution and complete chlorine substitution), a cyano group and a nitro group.

Two or more R^{1 8} (s) may be present, R^{1 8} (s) may be crosslinked to each other, and entire R^{1 8} may form a cyclic structure (an aromatic group). R^{1 8} may be substituted with the above substituents exemplified as for R^{1 8}. R^{1 8} may be interrupted with an oxygen atom, a sulfur atom, a nitrogen atom, a carbonyl group, an ester bond, etc.

The method for generating (or producing) a highly reactive intermediate according to the embodiment of the present invention comprises preparing a piezoelectric material (3).

In the embodiment of the present invention, the piezoelectric material (3) is not particularly limited as long as it is a material having an ability to generate a voltage when a mechanical strain is applied (piezoelectricity), and can generate a highly reactive intermediate.

The piezoelectric material (3) can include, for example, barium titanate, strontium titanate, lithium niobate, tourmaline, quartz, topaz, sucrose, Rochelle salt (KNaC₄ H₄ O₆ ·4H₂O), gallium orthophosphate (GaPO₄ ), langasite (La₃ SiO_{1 4} ), lead titanate (PbTiO₃ ), lead zirconate titanate, potassium niobate (KNbO₃ ), lithium tantalate (LiTaO₃ ), sodium tungstate (NaXWO₃ ), zinc oxide (ZnO, Zn₂ O₃ ), Ba₂ NaNb₅ O₅, Pb₂ KNb₅ O_{1 5} , lithium tetrabolate (Li₂ B₄ O₇ ), sodium potassium niobate ((K,Na)NbO₃), bismuth ferrite (BiFeO₃ ), sodium niobate (NaNbO₃ ), bismuth titanate (Bi₄ Ti₃ O_{1 2} ), sodium bismuth titanate (Na_{0 . 5} Bi_{0 . 5} TiO₃ ), polyvinylidene fluoride, aluminum nitride (AIN), gallium phosphate (GaPO₄), gallium arsenic (GaAs), etc.

The piezoelectric material (3) may be preferably titanates such as barium titanate, strontium titanate, lead titanate, bismuth titanate, sodium bismuth titanate and lead zirconate titanate; niobates such as lithium niobate, sodium niobate, potassium niobate and sodium potassium niobate; zinc oxide, etc.

The method for generating (or producing) a highly reactive intermediate according to the embodiment of the present invention comprises applying mechanical strain to the piezoelectric material (3) in the presence of the electron-accepting active compound (1) and the piezoelectric material (3), and subjecting the compound (1) to one-electron reduction to generate a corresponding highly reactive intermediate.

In the embodiment of the present invention, the reaction of subjecting the compound (1) to one-electron reduction to generate a corresponding highly reactive intermediate can be performed in the presence of the compound (1) and the piezoelectric material (3) (preferably by shaking to apply mechanical strain).

The compound (1) may be solid at 40°C.

In the present disclosure, the solvent may or may not be used, and the presence or absence of the solvent can be appropriately selected. It is possible to appropriately use solvents which are usually used in a (photocatalytic) redox reaction (e.g., aromatic solvents such as benzene, toluene, xylene and mesitylene; ether-based solvents such as diethyl ether, diisopropyl ether, dibutyl ether, tetrahydrofuran, dimethoxyethane and 1,4-dioxane; alcohol-based solvents such as methanol, ethanol and t-butanol; and polar solvents such as acetonitrile, dimethylformamide and dimethylacetamide) as necessary. The reaction can also be performed substantially without positively using the solvent.

In the embodiment of the present invention, the solvent may be allowed to be present in an amount of, for example, 0.01 to 3 microL/mg based on the total mass of the compound (1) and the piezoelectric material (3).

The generation of the highly reactive intermediate (mixing temperature) is usually performed at room temperature (e.g., 5 to 40°C), and can be performed by heating as appropriate.

It is possible to use, as the mixing method, any method which is capable of mixing such as shaking, rubbing, pressing, dispersing, kneading and crushing, and applying mechanical strain to the piezoelectric material (3), and the mixing method (or the method of applying mechanical strain) is not particularly limited as long as the method for producing a highly reactive intermediate of the present disclosure can be performed.

Mixing can be performed using, as such apparatus, for example:
crushers such as a ball mill, a rod mill, a jet mill and a SAG mill;
grinders such as a rotary stone mill and a bud crusher;
(horizontal axis rotation) container rotation type mixers such as horizontal cylindrical type, V type, double cone type, square cube type, S type and continuous V type mixers;
container rotation type mixers (with baffle plate blade) such as horizontal cylinder type, V type, double cone type and ball mill type mixers;
(rotary vibration) container rotary type mixers such as locking type and cross-rotary type mixers;
(horizontal axis rotation) fixed container type mixers such as ribbon type, paddle type, single shaft rotor type and bug mill type mixers;
(vertical axis rotation) fixed container type mixers such as ribbon type, screw type, planet type, turbine type, high-speed fluid type, rotating disk type and Marler type mixers;
(vibration) fixed container type mixers such as a vibration mill type mixer and a sieve;
(fluidized) fluid motion type mixers such as a non-uniform fluidized bed, a swirl fluidized bed, riser pipe type and jot pump type mixers; and
(gravity) fluid motion type mixers such as a gravity type mixer and a static mixer. As long as the reaction proceeds, the method and the apparatus used are not particularly limited. Regarding the mixer, it is possible to refer to, for example, Sakashita "Powder Mixing Process Technology", Coloring Material, 77(2), 75-85(2004), Table 5 and FIG. 9.

The mixing rate can also be appropriately selected.

The mixing time can also be appropriately selected. In the embodiment of the present invention, the mixing time can be, for example, 15 minutes or more, 30 minutes or more, 45 minutes or more, 60 minutes or more, 2 hours or less, 3 hours or less, 5 hours or less, or 10 hours or less.

The amount of the piezoelectric material (3) is not particularly limited as long as it is an amount which enables the generation of a highly reactive intermediate. For example, the piezoelectric material is allowed to exist in an amount of 0.5 mol% or more and 1,500 mol% or less on the basis (100%) of the number of mols obtained by multiplying the number of mols of the compound (1) by a valence.

In the embodiment of the present invention, the highly reactive intermediate thus produced is commonly used directly for the (mechano)redox reaction mentioned later.

The present invention provides, in another aspect, a (mechano)redox reaction method including the method for generating (or producing) a highly reactive intermediate, the redox reaction method comprising:
subjecting the highly reactive intermediate to a (mechano)redox reaction to produce a (mechano)redox reaction product.

The highly reactive intermediate obtained by the above production method is capable of undergoing a redox reaction by itself to give a redox reaction product, or undergoing a redox reaction with other compounds to give a redox reaction product. Any redox reaction can be selected in consideration of the highly reactive intermediate and the electron-accepting active compound (1) which generates it.

In the embodiment of the present invention, a redox reaction method is provided, which method comprises:
preparing an electron-accepting active compound (1);
preparing a piezoelectric material (3); and
applying mechanical strain to the piezoelectric material (3) in the presence of the electron-accepting active compound (1) and the piezoelectric material (3), and subjecting the compound (1) to one-electron reduction to generate a corresponding highly reactive intermediate, followed by a redox reaction of the highly reactive intermediate by itself to produce a redox reaction product.

The electron-accepting active compound (1), the piezoelectric material (3), the reaction conditions (e.g., reaction time, reaction temperature, quantitative relationship, apparatus) and the like of the redox reaction method can refer to those mentioned in the method for producing a highly reactive intermediate.

In the embodiment of the present invention, the redox reaction of the electron-accepting active compound (1) by itself can comprise that the activated fatty acid represented by the formula (I-4) as the electron-accepting active compound (1) undergoes a redox reaction by itself.

The above-mentioned compound can be referred to as the activated fatty acid represented by the formula (I-4).

For example, when the activated fatty acid represented by the formula (I-4) undergoes a redox reaction by itself, a compound having a double bond (e.g., an olefin) can be obtained by decarboxylation, as shown by the following formula: 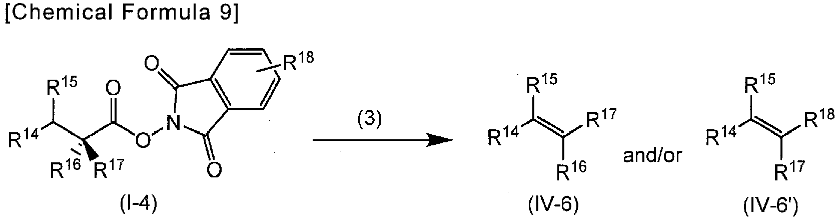 [wherein (I-4), R^{1 4} to R^{1 8} and (3) are as mentioned above].

In the embodiment of the present invention, the highly reactive intermediate is capable of undergoing a redox reaction with a compound (2) capable of reacting with it (i.e., serving as a reaction substrate) to produce a redox reaction product. For example, it is possible to exemplify, as the compound (2), a reaction substrate capable of reacting with a highly reactive intermediate selected from a radical, an anionic radical and an anion. Examples of such compound (2) include an aromatic compound optionally containing a hetero atom (2-1), a diboronic acid ester (2-2) and an aliphatic alcohol (2-3).

Therefore, in the embodiment of the present invention a method for generating a highly reactive intermediate is provided, which method comprises: preparing an electron-accepting active compound (1); preparing a piezoelectric material (3); preparing a compoud (2); and
applying mechanical strain to the piezoelectric material (3) in the presence of the compound (2), in addition to the electron-accepting active compound (1) and the piezoelectric material (3), and subjecting the compound (1) to one-electron reduction to generate a corresponding highly reactive intermediate, followed by a reaction of the highly reactive intermediate reacting with the compound (2) to produce a redox reaction product.

In the embodiment of the present invention, a redox reaction method is provided, which method comprises: further preparing at least one compound (2) selected from an aromatic compound (2-1) optionally containing a heteroatom, a diboronic acid ester (2-2) and an aliphatic alcohol (2-3); and
applying mechanical strain to the piezoelectric material (3) in the presence of the compound (2), in addition to the electron-accepting active compound (1) and the piezoelectric material (3), and subjecting the compound (1) to one-electron reduction to generate a corresponding highly reactive intermediate, followed by a rection of the highly reactive intermediate with the compound (2) to produce a redox reaction product.

In the redox reaction method between the electron-accepting active compound (1) and the compound (2), for example, it is possible to form a chemical bond selected from a C-B bond, a C-C bond, a C-Br bond and a C-I bond.

The redox reaction method according to the embodiment of the present invention comprises:
preparing at least one compound (2) selected from an aromatic compound (2-1) optionally containing a heteroatom, a diboronic acid ester (2-2) and an aliphatic alcohol (2-3) .

In the embodiment of the present invention, the aromatic compound (2-1) optionally containing a heteroatom, the diboronic acid ester (2-2) and the aliphatic alcohol (2-3) are not particularly limited as long as the objective redox reaction of the present invention can be performed..

In the embodiment of the present invention, the compound (2) is preferably selected from, for example:
an aromatic compound (2-1) optionally containing a heteroatom represented by the following general formula (II-1):

   A² -H

   [wherein A² is selected from an optionally substituted aryl group and an optionally substituted heteroaryl group];
a diboronic acid ester (2-2) represented by the following general formula (II-2): 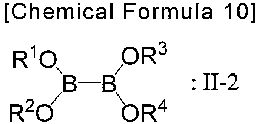 [wherein R¹ to R⁴ are each independently selected from hydrogen, an optionally substituted alkyl group and an optionally substituted aryl group, R¹ and R² may be bonded to each other, and R³ and R⁴ may be bonded to each other]; and
an aliphatic alcohol (2-3) represented by the following general formula (II-3): 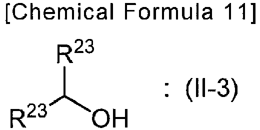 [wherein R^{2 3} may be the same or different from each other, and are each independently selected from hydrogen, an optionally substituted alkyl group, an optionally substituted alkenyl group and an optionally substituted alkynyl group, optionally substituted aryl group, and R^{2 3} and R^{2 3} may be bonded to each other].

In the embodiment of the present invention, the aromatic compound (2-1) optionally containing a heteroatom is not particularly limited as long as the redox reaction proceeds, more specifically, in the general formula (11-1),
the optionally substituted aryl group (or an aromatic hydrocarbon group) as for A² can include, for example, a phenyl group, a naphthyl group, an anthracenyl group (or an anthracene group), a phenanthrenyl group (or a phenanthrene group), a biphenyl group, a terphenyl group, a pyrenyl group (or a pyrene group), a perylenyl group (or a perylene group), a triphenylenyl group (or a triphenylene group) and a coronyl group (or a coronene group).

It is preferable to include a phenyl group, a naphthyl group, an anthracenyl group, a phenanthrenyl group, a biphenyl group, a terphenyl group, a pyrenyl group, a perylenyl group, a triphenyleny group and a coronyl group.

In the general formula (11-1),
the optionally substituted heteroaryl group (or a heteroaromatic group) as for A² preferably includes, for example:
sulfur-containing heteroaryl groups such as a thiophenyl group (a thiophene group or a thienyl group), a benzothienyl group and a dibenzothienyl group;
oxygen-containing heteroaryl groups such as a furanyl group (or a furan group), a benzofuranyl group, a dibenzofuranyl group, a phenyldibenzofuranyl group and a dibenzofuranylphenyl group;
nitrogen-containing heteroaryl groups such as a pyronyl group (or a pyrrol group), a benzopyronyl group, a dibenzopyronyl group, a pyridyl group (or a pyridine group), a pyrimidinyl group (or a pyrimidine group), a pyrazyl group (or a pyrazine group), a quinolyl group (or a quinoline group), an isoquinolyl group (or an isoquinoline group), a carbazolyl group (or a carbazole group), a 9-phenylcarbazolyl group, an acridinyl group (or an acridine group), a quinazolyl group (or a quinazoline group), a quinoxalyl group (or a quinoxaline group), a 1,6-naphthyldinyl group, a 1,8-naphthyldinyl group and a porphyrin group (or a porphyrin ring); and
heteroaryl groups containing two or more heteroatoms (e.g., nitrogen and sulfur), such as a benzothiazolyl group (or a benzothiazole group).

It is preferable to include a thiophenyl group, a furyl group, a pyronyl group, a benzthiophenyl group, a benzfuranyl group, a benzpyronyl group, a dibenzthiophenyl group, a dibenzfuranyl group and a dibenzpyronyl group.

The substituent, with which the aryl group and the heteroaryl group can be substituted, is not particularly limited as long as the objective redox reaction of the present invention can be performed.

The substituent includes, for example:
alkyl groups having 1 to 24 carbon atoms, for example, 1 to 18 carbon atoms, for example, 1 to 12 carbon atoms, for example, 1 to 8 carbon atoms (e.g., a methyl group, an ethyl group, an n-propyl group, an isopropyl group, an n-butyl group, an isobutyl group, a tert-butyl group, a pentyl group, a hexyl group, an octyl group, etc.),
alkoxy groups having 1 to 24 carbon atoms, for example, 1 to 18 carbon atoms, for example, 1 to 12 carbon atoms, for example, 1 to 8 carbon atoms (e.g., a methoxy group, an ethoxy group, an n-propoxy group, an isopropoxy group, an n-butoxy group, an isobutoxy group, a tert-butoxy group, a pentyloxy group, a hexyloxy group, an octyloxy group, etc.),
cycloalkyl groups having 3 to 24 carbon atoms, for example, 3 to 18 carbon atoms, for example, 3 to 12 carbon atoms, for example, 3 to 8 carbon atoms (e.g., a cyclopropyloxy group, a cyclobutyloxy group, a cyclopentyloxy group, a cyclohexyloxy group, etc.),
alkenyl groups having 1 to 24 carbon atoms, for example, 1 to 18 carbon atoms, for example, 1 to 12 carbon atoms, for example, 1 to 8 carbon atoms (e.g., an ethenyl group, a propenyl group, a butenyl group, a pentenyl group, a hexenyl group, an octenyl group, etc.),
alkynyl groups having 1 to 24 carbon atoms, for example, 1 to 18 carbon atoms, for example, 1 to 12 carbon atoms, for example, 1 to 8 carbon atoms (e.g., an ethynyl group, a propynyl group, a butynyl group, a pentynyl group, a hexynyl group, an octynyl group, etc.),
aryl groups having 5 to 24 carbon atoms, for example, 5 to 18 carbon atoms, for example, 5 to 12 carbon atoms, for example, 5 to 8 carbon atoms (e.g., a phenyl group, a naphthyl group, a biphenyl group, etc.),
aryloxy groups having 5 to 24 carbon atoms, for example, 5 to 18 carbon atoms, for example, 5 to 12 carbon atoms, for example, 5 to 8 carbon atoms (e.g., a phenoxy group, a naphthyloxy group, a biphenyloxy group, etc.),
heteroaryl groups having 4 to 24 carbon atoms, for example, 1 to 18 carbon atoms, for example, 1 to 12 carbon atoms, for example, 1 to 8 carbon atoms (e.g., a thiophenyl group, a furanyl group, a carbazole group, a benzothiophenyl group, a benzofuranyl group, an indolyl group, a pyrrolyl group, a pyridyl group, etc.),
acyl groups having 1 to 24 carbon atoms, for example, 1 to 18 carbon atoms, for example, 1 to 12 carbon atoms, for example, 1 to 8 carbon atoms (e.g., an acetyl group, a propionyl group, a butanoyl group, a pentanoyl group, a heptanoyl group, a group in which a carbonyl group included in the acyl group is substituted with an ester group or an amide group, etc.),
alkoxycarbonyl groups having 1 to 24 carbon atoms, for example, 1 to 18 carbon atoms, for example, 1 to 12 carbon atoms, for example, 1 to 8 carbon atoms (e.g., a methoxycarbonyl group, an ethoxycarbonylcarbonyl group, a propoxycarbonyl group, a butoxycarbonyl group, a tert-butoxycarbonyl group, a pentoxycarbonyl group, etc.),
amino groups having 1 to 24 carbon atoms, for example, 1 to 18 carbon atoms, for example, 1 to 12 carbon atoms, for example, 1 to 8 carbon atoms (e.g., a diphenylamino group, a dimethylamino group, etc.), and
fluorine (including partial fluorine substitution and complete fluorine substitution), a cyano group and a nitro group.
The substituents may be crosslinked to each other, and the entire substituent may form a cyclic structure (an aromatic group). Further, the above-mentioned substituent may be further substituted with the above-mentioned substituent.

More specifically, it is possible to exemplify, as the optionally substituted aryl compound represented by the general formula (II-1) as for A², for example, the following groups:
naphthyl groups such as a naphthyl group and an aryl (e.g., phenyl, etc.) naphthyl groups;
phenanthrenyl groups;
anthracenyl groups such as an anthracenyl group, an aryl (e.g., phenyl, etc.) anthracenyl group and a diaryl (e.g., dinaphthyl, etc.) anthracenyl group;
pyrenyl groups such as a pyrenyl group and an alkyl (e.g., t-butyl, etc.) pyrenyl group;
coronyl groups such as a coronyl group and an alkyl (e.g., t-butyl, etc.) coronyl group;
biphenyl groups such as a biphenyl group and a biphenyl group having an alkylene (e.g., propylene, isopropylene, etc.) crosslink;
phenyl groups such as a phenyl group, an alkyl (e.g., methyl, t-butyl) phenyl group, a dialkyl (e.g., dimethyl) phenyl group, an alkoxy (e.g., methoxy) phenyl group, a dialkylamino (e.g., dimethylamino) phenyl group, a diaryl (e.g., diphenyl) aminophenyl group, a perfluoroalkyl (e.g., trifluoromethyl) phenyl group, an alkyl (e.g., ethyl)oxycarbonylphenyl group, an alkanoyl (e.g., acyl) phenyl group, a fluorophenyl group, chlorophenyl group, a bromophenyl group, a nitrophenyl group and a cyanophenyl group;
aryl (e.g., phenyl, etc.) substituted carbazolyl groups;
anthracene-9.10-dione groups;
thienyl groups such as a thienyl group, an aryl (e.g., phenyl, etc.) substituted thienyl group, an alkylsubstituted thienyl group and an alkoxycarbonyl (e.g., methoxycarbonyl, tert-butoxycarbonyl group, etc.) substituted thienyl group;
furanyl groups such as a furanyl group, an aryl (e.g., phenyl, etc.) substituted furanyl group, an alkyl substituted furanyl group and an alkoxycarbonyl (e.g., methoxycarbonyl, tert-butoxycarbonyl group, etc.) substituted furanyl group;
pyronyl groups such as a pyronyl group, an aryl (e.g., phenyl, etc.) substituted pyronyl group, an alkyl substituted pyronyl group and an alkoxycarbonyl (e.g., methoxycarbonyl, tert-butoxycarbonyl group, etc.) substituted pyronyl group; and
benzpyronyl groups such as a benzpyronyl group, an aryl (e.g., phenyl, etc.) substituted benzpyronyl group, an alkyl substituted benzpyronyl group and an alkoxycarbonyl (e.g., methoxycarbonyl, tert-butoxycarbonyl group, etc.) substituted benzpyronyl group.

It is possible to use, as the optionally substituted aryl compound (an aromatic compound), commercially available compounds.

In the embodiment of the present invention, the diboronic acid ester (2-2) is not particularly limited as long as it has a B-B bond and undergoes a redox reaction with the above electron-accepting active compound (1) to give a product in which a B-C bond is formed.

In the embodiment of the present invention, the diboronic acid ester (2-2) includes a diboronic acid mono ester and a diboronic acid, for example, tetrahydroxydiboran.

The diboronic acid ester (2-2) includes a diboronic acid alkyl ester, a diboronic acid alkylene glycol ester, a diboronic acid aryl ester, a diboronic acid arylene glycol ester and tetrahydroxydiboran.

In the embodiment of the present invention, more specifically, the diboronic acid ester (including an ester and an acid) (2-2) can be represented, for example, by:
the general formula (II-2): 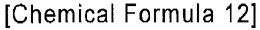 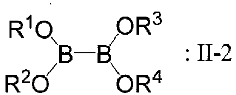 [wherein R¹ to R⁴ are each independently selected from hydrogen, an optionally substituted alkyl group and an optionally substituted aryl group (or an aromatic hydrocarbon group), R¹ and R² may be bonded to each other, and R³ and R⁴ may be bonded to each other].

In the formula (II-2), R¹ to R⁴ are each independently selected from hydrogen, an optionally substituted alkyl group and an optionally substituted aryl group, R¹ and R² may be bonded to each other, and R³ and R⁴ may be bonded to each other. Further, R¹ and R² may form a cyclic structure together, and R³ and R⁴ may form a cyclic structure together. The cyclic structure may be an aromatic group. Examples thereof include a 1,2-phenylene group, etc.

The optionally substituted alkyl group includes, for example, a methyl group, an ethyl group, a propyl group, an isopropyl group, a butyl group, an isobutyl group, a t-butyl group, etc. Further, two alkyl groups may be bonded, and R¹ -R² and R³ -R⁴ include, for example, an ethylene group, a 1,1,2,2-tetramethylethylene group, a 2,2-dimethylpropylene group, a hexylene group (or a 1,1,3-trimethylpropylene group), etc.

The optionally substituted aryl group is selected, for example, from a phenyl group, a naphthyl group, a biphenyl group, etc.

It is possible to select the substituent, with which the alkyl group and the aryl group may be substituted, form an alkyl group, an aryl group, an alkoxy group, an aryloxy group, etc. The substituents may be crosslinked to each other. The substituent may be further substituted with a substituent.

More specifically, the diboronic acid ester includes, for example, bis(pinacolato)diboron, bis(neopentyl glycolate)diboron, bis(hexylene glycolato)diboron, bis(catecholato)diboron), etc.

It is possible to use, as the diboronic acid ester (2-2), commercially available products.

In the present disclosure, the aliphatic alcohol (2-3) means that the hydroxyl group is bonded to the carbon atom of an aliphatic group, and the aliphatic group may be chain or cyclic, and may or may not be substituted. The aliphatic group may be interrupted with a heteroatom such as oxygen, nitrogen or sulfur, or a functional group such as a carbonyl group, an ester group or an amide group, and there is no particular limitation as long as the objective redox reaction of the present invention can be performed.

In the embodiment of the present invention, more specifically, the aliphatic alcohol (2-3) can be represented, for example, by:
the following general formula (II-3): 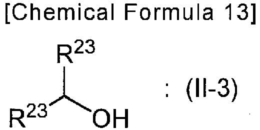 [wherein R^{2 3} may be the same or different from each other, and are each independently selected from hydrogen, an optionally substituted alkyl group, an optionally substituted alkenyl group, an optionally substituted alkynyl group and an optionally substituted aryl group, and R^{2 3} and R^{2 3} may be bonded to each other].

The optionally substituted alkyl group includes, for example, a methyl group, an ethyl group, a propyl group, an isopropyl group, a butyl group, an isobutyl group, a t-butyl group, a pentyl group, a hexyl group, an octyl group, etc.

The optionally substituted alkenyl group includes, for example, an ethenyl group, a propenyl group, an isopropenyl group, a butenyl group, an isobutenyl group, a pentenyl group, etc.

The optionally substituted alkynyl group includes, for example, an acetylenyl group, a propynyl group, a butynyl group, a pentynyl group, a hexynyl group, etc.

Further, two R^{2 3} (s) may be bonded to form a cyclic structure.

The optionally substituted aryl group may be selected, for example, from a phenyl group, a naphthyl group, a biphenyl group, etc.

The above-mentioned substituents may be selected from an alkyl group, an aryl group, an alkoxy group, an aryloxy group, an ester group, a carbonyl group, an amino group, a nitro group, a cyano group, fluorine, chlorine, etc. R^{2 3} (s) may be crosslinked to each other to form a ring. The substituent may be further substituted. The alkyl group, the alkenyl group, the alkynyl group, etc. as for R^{2 3} may be interrupted with oxygen, nitrogen, sulfur atom, etc.

It is possible to exemplify, as the aliphatic alcohol, for example, a primary aliphatic alcohol and a secondary aliphatic alcohol.

In the embodiment of the present invention, the redox reaction between the electron-accepting active compound (1) and the compound (2) can include, for example, the following reactions:
a redox reaction between an aryl compound having a leaving group represented by the formula (1-1) as the electron-accepting active compound (1), and an aromatic compound (2-1) optionally containing a heteroatom or a diboronic acid ester (2-2) as the compound (2);
a redox reaction between trifluoromethyl compounds represented by the formulas (I-2a) to (I-2c) as the electron-accepting active compound (1), and an aromatic compound (2-1) optionally containing a heteroatom as the compound (2); and
a redox reaction between a bromide or iodide represented by the formula (I-3) as the electron-accepting active compound (1) and an aliphatic alcohol (2-3) as the compound (2).

For example, when an aryl compound having a leaving group represented by the formula (1-1) undergoes a redox reaction with an aromatic compound (2-1) optionally containing a heteroatom, it is possible to obtain a compound in which the aromatic ring of the aryl compound having a leaving group is directly bonded to the aromatic ring of the aromatic compound (2-1) optionally containing a heteroatom.

For example, the compound is represented by the following formula: 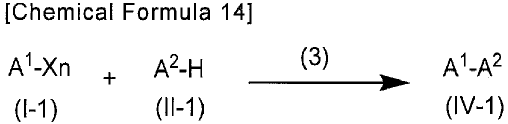 [wherein (1-1), A¹ , X, n, (II-1), A² and (3) are as mentioned above].

For example, when an aryl compound having a leaving group represented by the formula (I-1) undergoes a redox reaction with a diboronic acid ester (2-2), it is possible to obtain a compound in which the aromatic ring of the aryl compound having a leaving group has a boronic acid ester as the substituent.

For example, the compound is represented by the following formula: 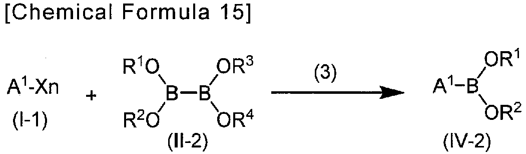 [wherein (1-1), A¹ , X, n, (II-2), R¹ to R⁴ and (3) are as mentioned above].

For example, when trifluoromethyl compounds represented by the formulas (I-2a) to (I-2c) undergo a redox reaction with an aromatic compound (2-1) optionally containing a heteroatom, it is possible to obtain a compound in which the aromatic compound (2-1) optionally containing a heteroatom is trifluoromethylated.

For example, the compound is represented by the following formula: 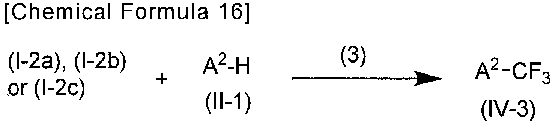 [wherein (I-2a) to (1-2c), (II-1), A² and (3) are as mentioned above].

For example, when a bromide or iodide represented by the formula (I-3) undergoes a redox reaction with an aliphatic alcohol (2-3), it is possible to obtain a compound in which the hydroxyl group of the aliphatic alcohol (2-3) is substituted with bromine or iodine: 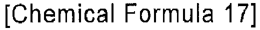 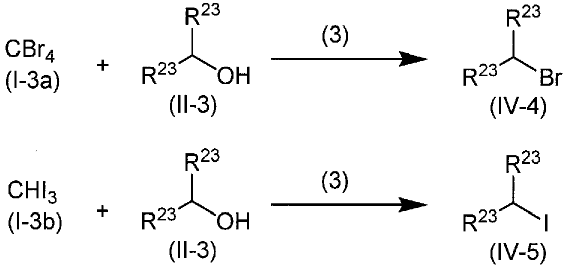 [wherein (I-3a) to (1-3b), (II-3), R^{2 3} and (3) are as mentioned above].

The redox reaction method according to the embodiment of the present invention comprises:
applying mechanical strain to the piezoelectric material (3) in the presence of the compound (2), in addition to the electron-accepting active compound (1) and the piezoelectric material (3), and subjecting the compound (1) to one-electron reduction to generate a corresponding highly reactive intermediate, followed by a reaction of the highly reactive intermediate with the compound (2) to produce a redox reaction product.

The electron-accepting active compound (1), the piezoelectric material (3), the reaction conditions (e.g., reaction time, reaction temperature, quantitative relationship, apparatus) and the like of the redox reaction method can refer to those mentioned in the method for producing a highly reactive intermediate.

In the embodiment of the present invention, the redox reaction between the compound (1) and the compound (2) can be performed in the presence of the piezoelectric material (3) (preferably by shaking to apply mechanical strain).

Either or both of the compound (1) and the compound (2) may be solid at 40°C.

In the present disclosure, the solvent may or may not be used, and the presence or absence of the solvent can be appropriately selected. It is possible to appropriately use solvents which are usually used in a redox reaction using a photocatalyst (e.g., aromatic solvents such as benzene, toluene, xylene and mesitylene; ether-based solvents such as diethyl ether, diisopropyl ether, dibutyl ether, tetrahydrofuran, dimethoxyethane and 1,4-dioxane; alcohol-based solvents such as methanol, ethanol and t-butanol; and polar solvents such as acetonitrile, dimethylformamide and dimethylacetamide) as necessary. The reaction can also be performed substantially without positively using the solvent.

In the embodiment of the present invention, the solvent may be allowed to be present in an amount of, for example, 0.01 to 3 microL/mg based on the total mass of the compound (1), the compound (2) and the piezoelectric material (3).

The redox reaction (mixing temperature) is usually performed at room temperature (e.g., 5 to 40°C), but can be performed by heating as appropriate.

It is possible to use, as the mixing method, any method which is capable of mixing such as shaking, rubbing, pressing, dispersing, kneading and crushing, and applying mechanical strain to the piezoelectric material (3), and the mixing method (or the method of applying mechanical strain) is not particularly limited as long as the objective redox reaction of the present disclosure can be allowed to proceed.

As such apparatus, the above-mentioned apparatuses can be used as a reference.

The mixing time can also be appropriately selected. In the embodiment of the present invention, the mixing time can be, for example, 15 minutes or more, 30 minutes or more, 45 minutes or more, 60 minutes or more, 2 hours or less, 3 hours or less, 5 hours or less, or 10 hours or less.

The equivalent ratio of the compound (1) to the compound (2) (compound (1)/compound (2)) is not particularly limited as long as it is an equivalent ratio which can allow the redox reaction to proceed, and may be, for example, 10/1 to 1/10.

The equivalent of the piezoelectric material (3) is not particularly limited as long as it is an amount which can allow the redox reaction to proceed, and the piezoelectric material may be allowed to exist in an amount of, for example, 0.5 mol% or more and 1,500 mol% or less on the basis (100%) of the number of mols obtained by multiplying the number of mols of the compound (1) by the valence.

The redox reaction product thus obtained can be appropriately purified. The purification method is not particularly limited as long as the redox reaction product can be purified. It is possible to use, as the purification method, for example, a conventional method such as recrystallization and column chromatography.

The present invention provides, in a further aspect, a method for producing a redox reaction product using a redox reaction method.

It is possible to directly apply the formulas and terms described in the redox reaction method to the method for producing a redox reaction product.

In the embodiment of the present invention, since the piezoelectric material (3) can be used repeatedly, the cost of the raw material can be reduced. The reaction can proceed with no solvent or with an appropriate solvent, so that an appropriate solvent can be used in an appropriate amount of the solvent.

In the embodiment of the present invention, reference can be made to the above-mentioned electron-accepting active compound (1), piezoelectric material (3), method (or mixing method) which applies mechanical strain, description of at least one compound (2) selected from the aromatic compound (2-1) optionally containing a heteroatom, the diboronic acid ester (2-2) and the aliphatic alcohol (2-3), description of the method for producing a highly reactive intermediate, description of the redox reaction method, etc. in each of the embodiments, mutually, as much as applicable.

The reaction method and the production method according to the embodiment of the present invention are considered to exert excellent effect by this reason, but such a reason does not limit the present invention in any way.

### [Examples]

The present invention will be described specifically in detail below by way of Examples and Comparative Examples. It should be understood that these Examples are merely embodiments of the present invention and the present invention is in no way limited by these Examples.

Compounds used in Examples were specifically exemplified in the following Examples.

Regarding compounds used in the Examples, such as an electron-accepting active compound (1), an aromatic compound (2-1) optionally containing a heteroatom, diboronic acid ester (2-2) and a piezoelectric material (3), commercially available products were directly used without purification

The redox reaction was performed at room temperature using a ball mill, Model MM400, manufactured by Retsch Co., Ltd. (changed company name to Verder Scientific Co., Ltd.).

### Example 1

In a 1.5 mL stainless steel ball mill jar containing stainless steel balls having a diameter of 5 mm, 4-chlorophenyldiazonium tetrafluoroborate (1a) (67.9 mg, 0.3 mmol, 1.0 equiv), furan (2-1a) (306.3 mg, 4.5 mmol, 15.0 equiv) and barium titanate (3a) (349.8 mg, 1.5 mmol, 5.0 equiv) were charged under the air. A lid of the ball mill jar was closed and the ball mill jar was attached to a ball mill (Model MM400, manufactured by Retsch Co., Ltd.), followed by shaking and stirring (30 Hz) for 60 minutes. After completion of the reaction, the reaction mixture was passed through short silica gel column chromatography with diethyl ether to remove the barium titanate and inorganic salt. After removing diethyl ether by an evaporator, the objective reaction product was isolated by purification using silica gel column chromatography (38.9 mg, 0.219 mmol, isolated yield of 73%).

The results of Example 1 are also shown in Table 1.

### Examples 2 to 7 and Comparative Example 1

Using the same method as in Example 1, except that barium titanate (3a) in Example 1 was replaced by each of strontium titanate (3b), lithium niobate (3c) and zinc oxide (3d), the stirring rate was replaced by 10 Hz or 20 Hz, the size of the ball mill jar was changed, and the ball size was changed, reactions of Examples 2 to 7 were performed to obtain reaction products. Using the same method as in Example 2, except that barium titanate (3a) in Example 1 was not used, a reaction of Comparative Example 1 was performed. The results of Examples 2 to 7 and Comparative Example 1 are shown in Table 1.

**[Table 1]**

| 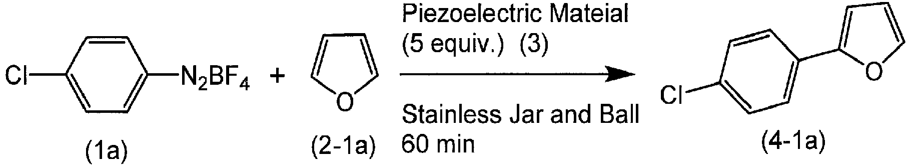 | | | | | | | | |
|---|---|---|---|---|---|---|---|---|
| Table 1 | Example | | | | | | | Comparative Example |
| | 1 | 2 | 3 | 4 | 5 | 6 | 7 | 1 |
| Piezoelectric material | BaTiO3 (3a) | BaTiO3 (3a) | BaTiO3 (3a) | SrTiO3 (3b) | LiNbO3 (3c) | ZnO (3d) | BaTiO3 (3a) | None |
| Stirring rate (Hz) | 30 | 20 | 10 | 20 | 30 | 30 | 30 | 30 |
| Jar size (mL) | 1.5 | 1.5 | 1,5 | 1,5 | 1.5 | 1.5 | 5.0 | 1.5 |
| Ball size (mm) | 5 | 5 | 5 | 5 | 5 | 5 | 7.5 | 5 |
| Yield (%) | 73 | 40 | 29 | 3 | 24 | 4 | 82 0 | 0 |

### Examples 7 to 18

Using the same method as in Example 7, except that 4-chlorophenyldiazonium tetrafluoroborate (1a) in Example 7 was replaced by various diazonium tetrafluoroborates (1b) to (1m), reactions of Examples 8 to 18 were performed to obtain reaction products. Just in case, the results of Example 7 are also shown in Table 2 together with those results.

**[Table 2]**

| | | | | |
|---|---|---|---|---|
| 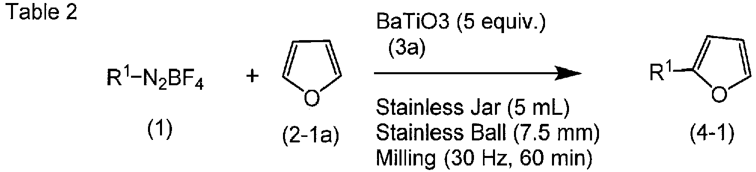 | | | | |
| Example | 7 | 8 | 9 | 10 |
| 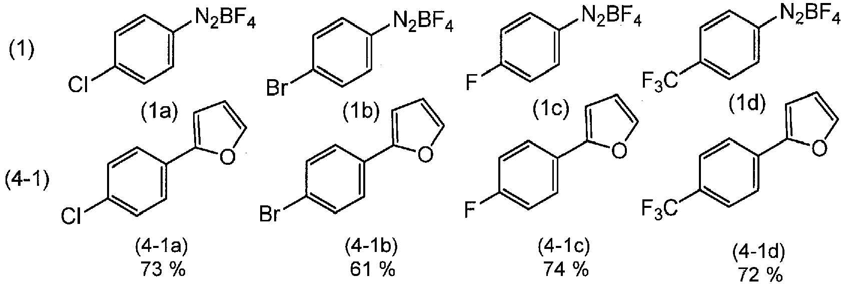 | | | | |
| Example | 11 | 12 | 13 | 14 |
| 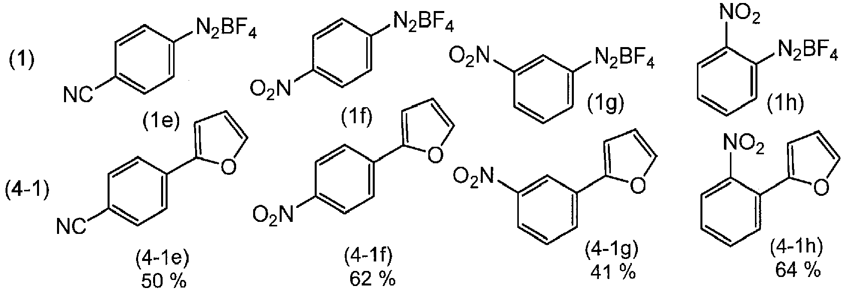 | | | | |
| Example | 15 | 16 | 17 | 18 |
| 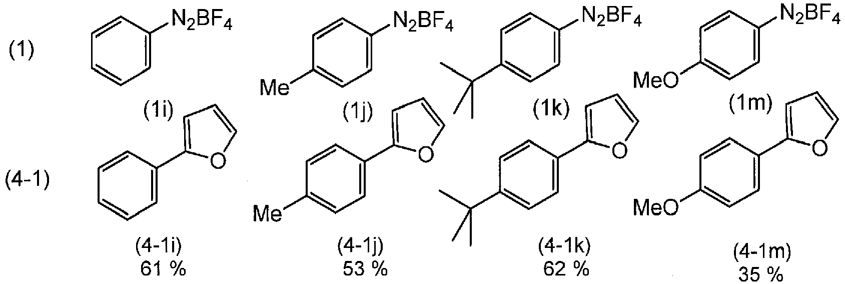 | | | | |

### Examples 21 to 24

Using the same method as in Example 7, except that 4-chlorophenyldiazonium tetrafluoroborate (1a) in Example 7 was replaced by various diazonium tetrafluoroborate (1f) to (1n) and heteroaromatic compounds (2-1b) to (2-1c) were used in addition to furan (2-1a), reactions of Examples 21 to 24 were performed to obtain reaction products. These results are shown in Table 3.

**[Table 3]**

| | | | | |
|---|---|---|---|---|
| Table 3 | 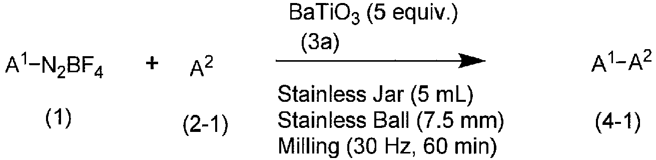 | | | |
| Example | 21 | 22 | 23 | 24 |
| 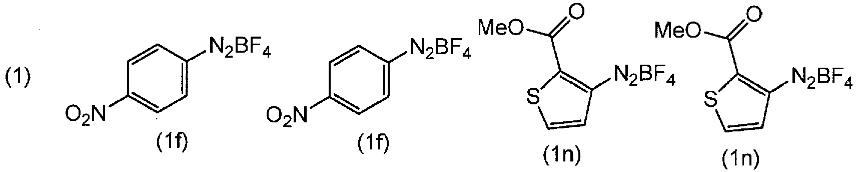 | | | | |
| 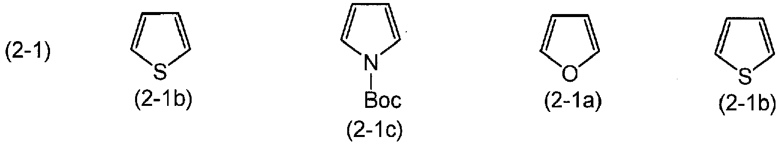 | | | | |
| 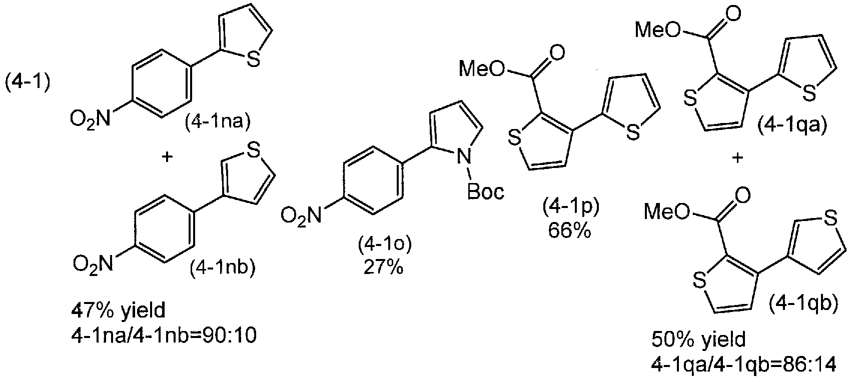 | | | | |

### Examples 27 to 28

Using the same method as in Example 7, except that 4-chlorophenyldiazonium tetrafluoroborate (1a) in Example 7 was replaced by 4-nitrophenyldiazonium tetrafluoroborate (1f), furan (2-1a) was replaced by polycyclic aromatic compounds (2-1d) to (2-1e), and acetonitrile was used in an amount of 0.12 µL per 1 mg of the total mass of all solid reactants used, reactions of Examples 27 to 28 were performed to obtain reaction products. These results are shown in Table 4.

**[Table 4]**

| | | |
|---|---|---|
| 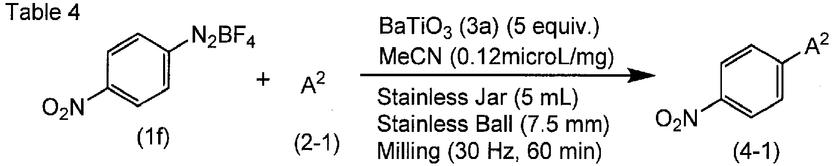 | | |
| Example | 27 | 28 |
| 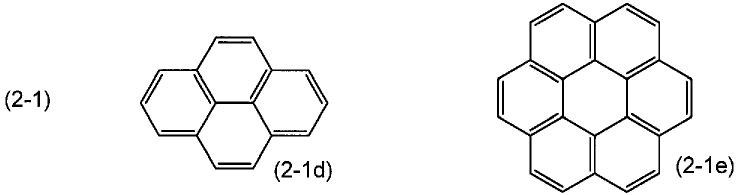 | | |
| 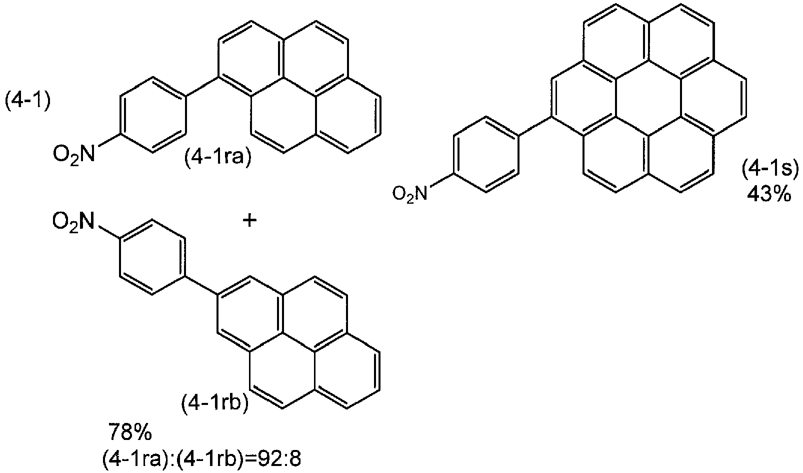 | | |

### Example 31

Using the same method as in Example 7, except that 4-chlorophenyldiazonium tetrafluoroborate (1a) in Example 7 was replaced by 4-tert-butylphenyldiazonium tetrafluoroborate (1k), BaTiO3 used was recovered after the reaction, the reaction was performed repeatedly using the recovered BaTiO3, and the reaction was performed repeatedly five times in total by recovering BaTiO3 for each reaction, a reaction of Example 31 was performed to obtain a reaction product. These results are shown in Table 5.

**[Table 5]**

| 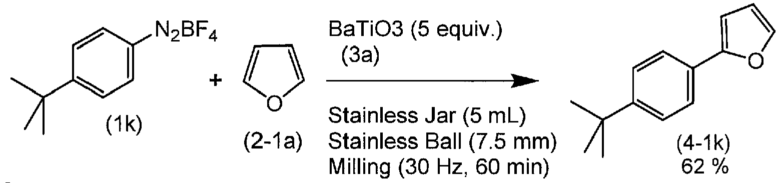 | | |
|---|---|---|
| Table 5 | | |
| Example 31 | (4-1)k Yield (%) | Recovery of BaTiO3 (3a) (%) |
| 1 st run | 73 | 95 |
| 2nd run | 71 | 96 |
| 3rd run | 66 | 96 |
| 4th run | 52 | 98 |
| 5th run | 43 | - |

### Example 41

In a 5 mL stainless steel ball mill jar containing stainless steel balls having a diameter of 7.5 mm, 4-chlorophenyldiazonium tetrafluoroborate (1a) (0.3 mmol, 1.0 equiv), bispinacolatodiboron (2-2a) (76.2 mg, 0.3 mmol, 1.0 equiv), barium titanate (3a) (349.8 mg, 1.5 mmol, 5.0 equiv) and acetonitrile (59 microL) (0.12 µL per 1 mg of the total mass of all solid reactants used) were charged under the air. A lid of the ball mill jar was closed and the ball mill jar was attached to a ball mill (Model MM400, manufactured by Retsch Co., Ltd.), followed by shaking and stirring (30 Hz) for 180 minutes. After completion of the reaction, the reaction mixture was passed through short silica gel column chromatography with diethyl ether to remove the barium titanate and inorganic salt. After removing diethyl ether by an evaporator, the objective borylated reaction product was isolated by purification using silica gel column chromatography (4-2a, isolated yield of 61%).

The results of Example 41 are also shown in Table 6.

### Examples 42 to 50

Using the same method as in Example 41, except that 4-chlorophenyldiazonium tetrafluoroborate (1a) in Example 42 was replaced by various diazonium tetrafluoroborates (1b) to (1m), reactions of Examples 42 to 50 were performed to obtain reaction products. The results of Examples 42 to 50 are shown in Table 6.

**[Table 6]**

| | | | | |
|---|---|---|---|---|
| 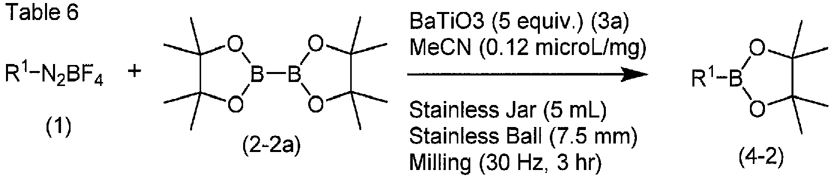 | | | | |
| Example | 41 | 42 | 43 | 44 |
| 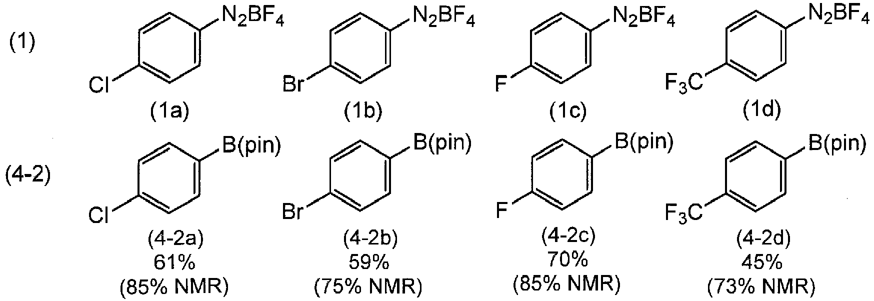 | | | | |
| Example | 45 | 46 | 47 | 48 |
| 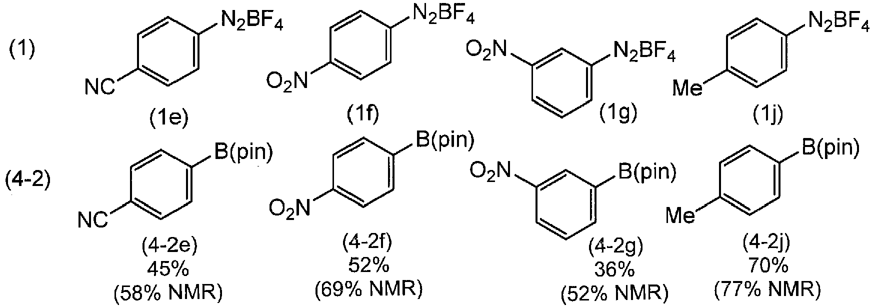 | | | | |
| Example | 49 | 50 | | |
| 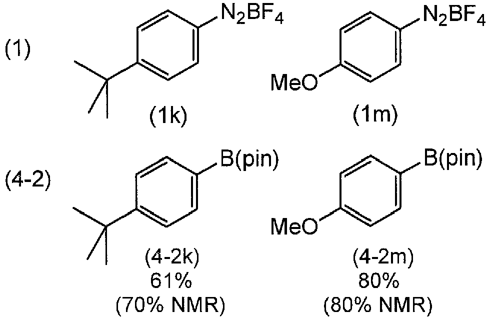 | | | | |

### Examples 51 to 58, Comparative Example 51

Using the same method as in Example 41, except that acetonitrile in Example 41 and its amount, and the stirring time were replaced by liquids shown in Table 7 and their amounts (0.20 µL per 1 mg of the total weight of all solid reactants used) and the stirring time, reactions of Examples 51 to 58 were performed to obtain reaction products. Using the same method as in Example 51, except that barium titanate (3a) in Examples 51 was not used, a reaction of Comparative Example 51 was performed. In Comparative Example 51, substantially no reaction product was obtained. The results of Examples 51 to 58 and Comparative Example 51 are shown in Table 7.

**[Table 7]**

| 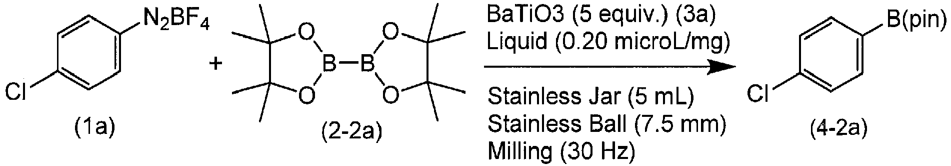 | | | | | | | | | |
|---|---|---|---|---|---|---|---|---|---|
| Table 7 | Example | | | | | | | | Comparative Example |
| | 51 | 52 | 53 | 54 | 55 | 56 | 57 | 58 | 51 |
| Piezoelectric material | BaTiO3 (3a) | BaTiO3 (3a) | BaTiO3 (3a) | BaTiO3 (3a) | BaTiO3 (3a) | BaTiO3 (3a) | BaTiO3 (3a) | BaTiO3 (3a) | None |
| Liquid (µL/mg) | MeCN 20 | MeCN 20 | MeCN 20 | DMF 20 | DMSO 20 | toluene 20 | hexane 20 | None 20 | MeCN 20 |
| Stirring rate (Hz) | 30 | 30 | 30 | 30 | 30 | 30 | 30 | 30 | 30 |
| Time (hour) | 3 | 1.5 | 1 | 1 | 1 | 1 | 1 | 1 | 3 |
| NMR vield(%) | 89 | 62 | 54 | 37 | 13 | 11 | 15 | 21 <1 | |

### Example 59

The same reaction as in Example 49 was performed, except that the process was performed by hitting 200 times using a hammer instead of using a ball mill at 30 Hz for 3 hours, a reaction product (4-2k) was obtained in a yield of 43% (NMR).

### Example 61

In a 1.5 mL stainless steel ball mill jar containing stainless steel balls having a diameter of 5 mm, 3-methyl-1H-indole (39.4 mg, 0.3 mmol, 1.0 equiv) (2-1f), 2,8-difluoro-5-(trifluoromethyl)-5H-dibenzo[b,d]thiophen-5-ium, trifluoromethanesulfonate (263.2 mg, 0.6 mmol, 2.0 equiv) (1-2a1) and barium titanate (349.8 mg, 1.5 mmol, 5.0 equiv) (3a) were charged under the air. A lid of the ball mill jar was closed and the ball mill jar was attached to a ball mill (Model MM400, manufactured by Retsch Co., Ltd.), followed by shaking and stirring (30 Hz) for 60 minutes. After completion of the reaction, the reaction mixture was passed through short silica gel column chromatography with ethyl acetate to remove the barium titanate and inorganic salt. After removing ethyl acetate by an evaporator, the objective reaction product (4-3a) was isolated by purification using silica gel column chromatography (31.7 mg, 0.159 mmol, isolated yield of 53%).

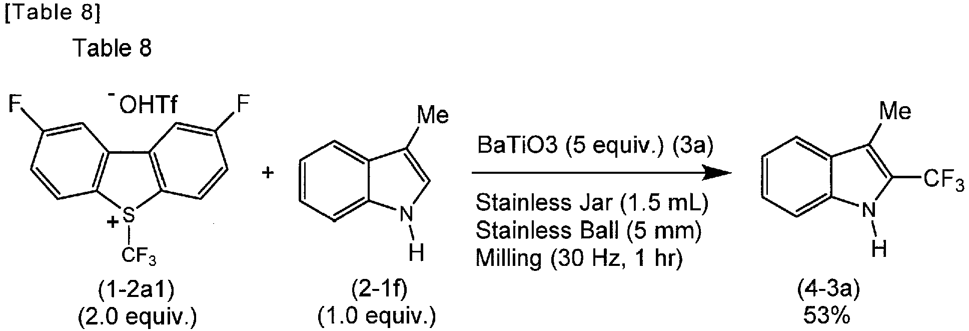

### Examples 62 to 68

In a 1.5 mL stainless steel ball mill jar containing stainless steel balls having a diameter of 5 mm, 3-methyl-1H-indole (0.6 mmol, 2.0 equiv) (2-1f), 2,8-difluoro-5-(trifluoromethyl)-5H-dibenzo[b,d]thiophen-5-ium trifluoromethanesulfonate (0.3 mmol, 1.0 equiv) (1-2a1) or 2,3,7,8-tetrafluoro-5-(trifluoromethyl)-5H-dibenzo[b,d]thiophen-5-ium trifluoromethanesulfonate (0.3 mmol, 1.0 equiv) (1-2a2), a piezoelectric material (1.5 mmol, 5.0 equiv) (3), and liquids (0.20 µL per 1 mg of the total mass of all solid reactants used) were charged under the air. A lid of the ball mill jar was closed and the ball mill jar was attached to a ball mill (Model MM400, manufactured by Retsch Co., Ltd.), followed by shaking and stirring (30 Hz) for 90 minutes. After completion of the reaction, crude products were analyzed by ^{1 9} F-NMR to determine the yield. The results are shown in Table 9.

### [Table 9]

**Table 9**

| 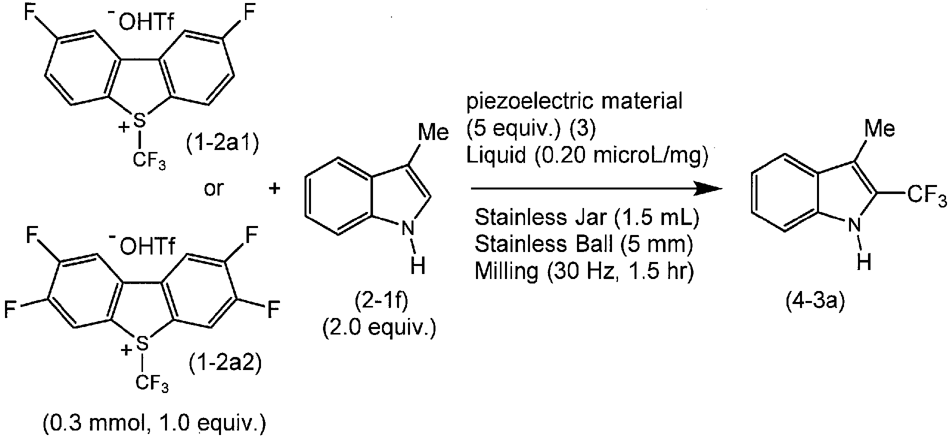 | | | | | | | |
|---|---|---|---|---|---|---|---|
| Table 9 | Example | | | | | | |
| | 62 | 63 | 64 | 65 | 66 | 67 | 68 |
| (I-2a) | (1-2a1) | (1-2a1) | (1-2a1) | (1-2a1) | (1-2a1) | (1-2a1) | (1-2a2) |
| Piezoelectric material (particle size) | BaTiO3 (<3 µm) | BaTiO3 (<3 µm) | BaTiO3 (<3 µm) | BaTiO3 (<75 µm) | LiNbO3 (<70 µm) | ZnO (<10 µm) | BaTiO3 (<3 µm) |
| Liquid | DMF | MeCN | Acetone | Acetone | Acetone | Acetone | Acetone |
| NMR yield (%) | 58 | 58 | 62 | 8 | 43 | 18 | 61 |

### Examples 69 to 72

Using the same method as in Example 64, except that the size of the stainless steel ball mill jar was changed, the size of the stainless steel balls was changed, and the shaking time and the shaking rate were changed, reactions of Examples 69 to 72 were performed. After completion of the reaction, crude products were analyzed by ^{1 9} F-NMR to determine the yield. The results are shown in Table 10.

### [Table 10]

**Table 10**

| 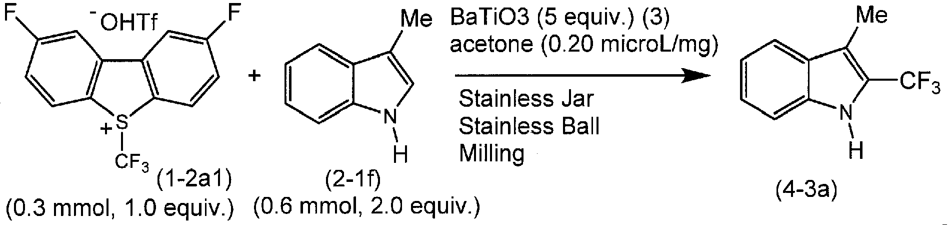 | | | | |
|---|---|---|---|---|
| Table 10 | Examples | | | |
| | 69 | 70 | 71 | 72 |
| Stainless Jar Size (mL) | 1.5 | 1.5 | 5 | 5 |
| Stainless Ball Size (mm) | 5 | 5 | 7.5 | 7.5 |
| Milling (Hz) | 30 | 25 | 30 | 30 |
| Milling (Time, hour) | 1.5 | 1.5 | 1.5 | 3 |
| NMR yield (%) | 62 | 19 | 73 | 80 (76)^{a} |

| | | | | |
|---|---|---|---|---|
| a) Isolated yield | | | | |

### Examples 73 to 84

Using the same method as in Example 72, except that 3-methyl-1H-indole (2.0 equiv.) (2-1f) was replaced by various aromatic compounds (2-1g) to (2-1q), reactions of Examples 73 to 84 were performed. After completion of the reaction, the reaction mixture was passed through short silica gel column chromatography with ethyl acetate or diethyl ether to remove the barium titanate and inorganic salt. After removing ethyl acetate or diethyl ether by an evaporator, the objective borylated reaction products were isolated by purification using silica gel column chromatography or gel permeation chromatography (GPC) to obtain the objective reaction product (4-3). After completion of the reaction, crude products were analyzed by ^{1 9} F-NMR to determine the NMR yield. The results are shown in Table 11. In Table 11, the yield represents an isolated yield, and the NMR yield is shown in parentheses. The CF3 product (4-3m) of Example 84 is a CF3-substituted melatonin.

**[Table 11]**

| | | | | |
|---|---|---|---|---|
| 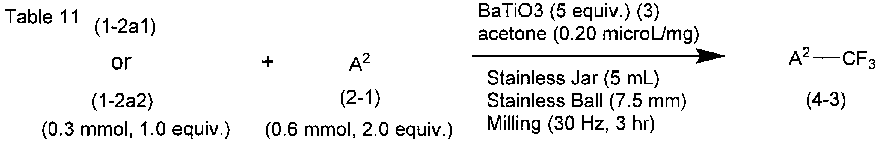 | | | | |
| Example | 73 | 74 | 75 | 76 |
| 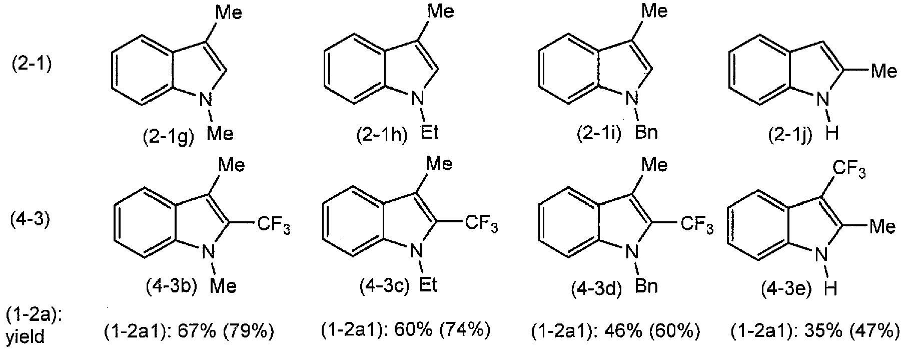 | | | | |
| Example | 77 | 78 | 79 | 80 |
| 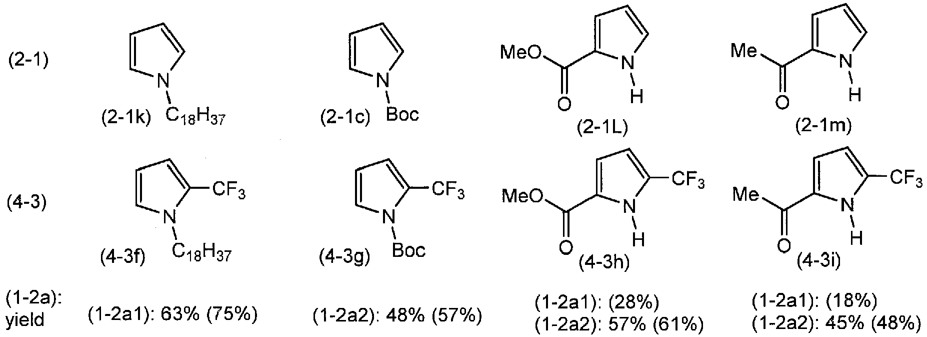 | | | | |
| Example | 81 | 82 | 83 | 84 |
| 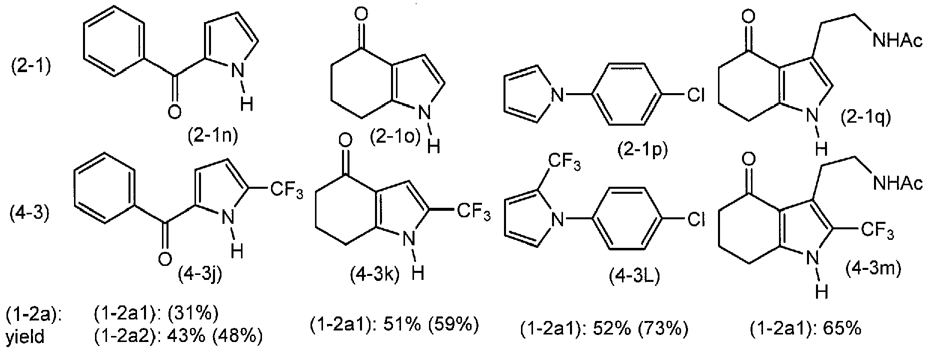 | | | | |

### Examples 85 to 91

Using the same method as in Examples 73 to 84, except that 3-methyl-1H-indole (2.0 equiv.) (2-1f) was replaced by various aromatic compounds (2-1r) to (2-1x), reactions of Examples 85 to 91 were performed. The results are shown in Table 12. In Table 12, the yield represents an isolated yield, and the NMR yield is shown in parentheses. Examples 85 to 87 are examples of electron-rich aromatic compounds, and Examples 88 to 91 are examples of tryptophan-containing peptides.

**[Table 12]**

| | | | |
|---|---|---|---|
| 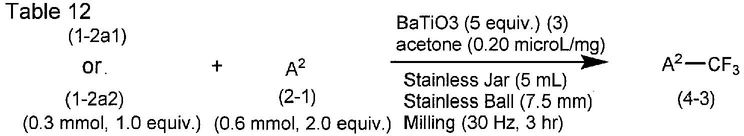 | | | |
| Example | 85 | 86 | 87 |
| 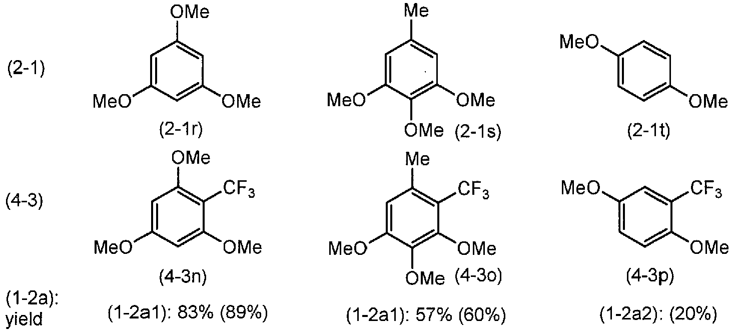 | | | |
| Example | 88 | | 89 |
| 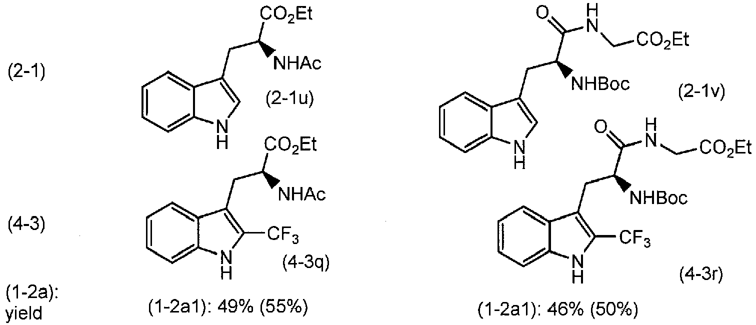 | | | |
| Example | 90 | | 91 |
| 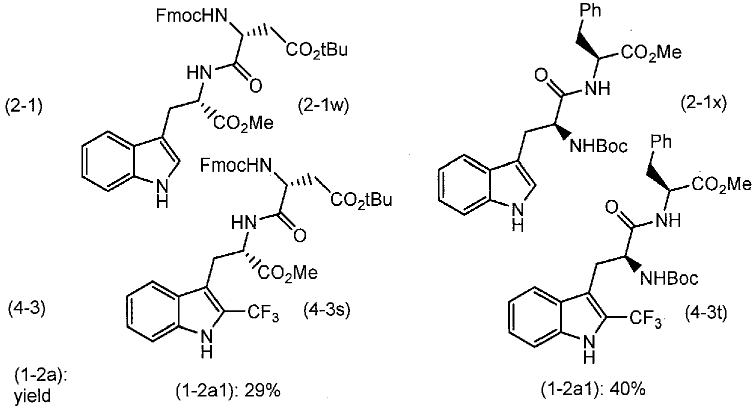 | | | |

### Example 92

The same reaction as in Example 64 was performed, except that 0.1 µL/mg of acetone was used and the process was performed by hitting 250 times using a hammer instead of using a ball mill at 30 Hz for 1.5 hours, a reaction product (4-2a) was obtained in a yield of 13% (NMR).

The redox reaction of Examples 1 to 92 comprises preparing an electron-accepting active compound (1); preparing at least one compound (2) selected from an aromatic compound (2-1) optionally containing a heteroatom and a diboronic acid ester (2-2); and subjecting a compound (1) to a redox reaction with a compound (2) in the presence of a piezoelectric material (3). Therefore, a chemical bond selected from C-B and C-C bonds can be formed, the amount of the solvent can be reduced, the reaction can be performed in the presence of the air, no light irradiation is required, and it is easier to scale up.

In both Comparative Examples 1 and 51, since the piezoelectric material (3) is not used, the redox reaction does not proceed.

### [Industrial Applicability]

According to the reaction method of the embodiment of the present invention, the amount of the solvent can be reduced, the reaction can be performed in the presence of the air, no light irradiation is required, and is easier to scale up.

### [Related Application]

This application claims priority under Article 4 of the Paris Convention of the Japanese Patent Law on Japanese Patent Application No. 2019-163323 filed on September 6, 2019, in Japan, the disclosure of which is incorporated by reference herein.

## Claims

1. A method for generating a highly reactive intermediate, which comprises:
preparing an electron-accepting active compound (1);
preparing a piezoelectric material (3); and
applying mechanical strain to the piezoelectric material (3) in the presence of the electron-accepting active compound (1) and the piezoelectric material (3), and subjecting the compound (1) to one-electron reduction to generate a corresponding highly reactive intermediate.

2. The method for generating a highly reactive intermediate according to claim 1, wherein the electron-accepting active compound (1) is selected from
an aryl compound having a leaving group represented by the following general formula (I-1):
A¹-Xn
wherein A¹ is selected from an optionally substituted aryl group and an optionally substituted heteroaryl group, X is a leaving group, and n is an integer of 1 or more, and
a trifluoromethyl compound selected from an optionally substituted trifluoromethyl-dibenzothiophene (I-2a), an optionally substituted trifluoromethyl-diphenylmercaptan (I-2b) and trifluoromethanesulfonyl chloride (I-2c) represented by the following formulas (I-2a) to (I-2c): 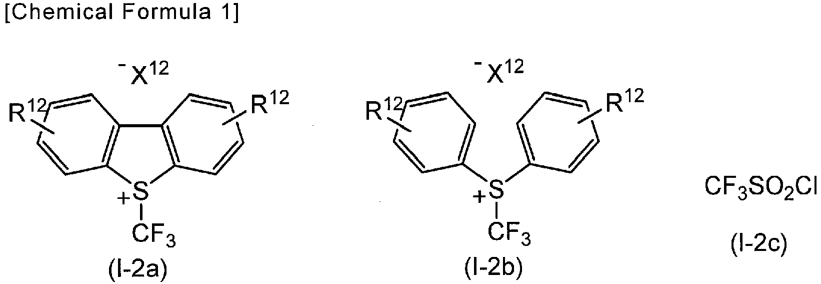 wherein R^{1 2} each independently comprises hydrogen, an alkyl group, an alkoxy group, a cycloalkyl group, an alkenyl group, an alkynyl group, an aryl group, an aryloxy group, an acyl group, an alkoxycarbonyl group, an amino group, fluorine, chlorine, a cyano group, a nitro group, etc., R^{1 2} (s) may be crosslinked to each other to form a cyclic structure, and also may have other substituents, R^{1 2} may be interrupted, for example, with an oxygen atom, a sulfur atom, a nitrogen atom, a carbonyl group, an ester bond, etc., and ⁻X^{1 2} represents an anion.

3. The method for generating a highly reactive intermediate according to claim 2, wherein the electron-accepting active compound (1) is selected from an aryl compound having a leaving group represented by the formula (I-1):
wherein, in the general formula (1-1), the optionally substituted aryl group as for A¹ comprises a phenyl group, a naphthyl group, an anthracenyl group, a phenanthrenyl group, a biphenyl group, a terphenyl group, a pyrenyl group, a perylenyl group and a triphenyleny group,
the optionally substituted heteroaryl group as for A¹ comprises a sulfur-containing heteroaryl group, an oxygen-containing heteroaryl group, a nitrogen-containing heteroaryl group, and a heteroaryl group containing two or more heteroatoms, and
the leaving group X comprises iodine, bromine, chlorine and a diazonium salt.

4. The method for generating a highly reactive intermediate according to any one of claims 1 to 3, wherein the piezoelectric material (3) comprises at least one selected from barium titanate, lithium niobate, tourmaline, quartz, topaz, sucrose, Rochelle salt (KNaC₄ H₄ O₆ ·4H₂ O), gallium orthophosphate (GaPO₄ ), langasite (La₃ Ga₅ SiO_{1 4} ), lead titanate (PbTiO₃ ), lead zirconate titanate, potassium niobate (KNbO₃), lithium tantalate (LiTaO₃ ), sodium tungstate (NaXWO₃ ), zinc oxide (ZnO, Zn₂ O₃ ), Ba₂ NaNb₅ O₅, Pb₂ KNb₅ O_{1 5} , lithium tetrabolate (Li₂ B₄ O₇ ), sodium potassium niobate ((K,Na)NbO₃ ), bismuth ferrite (BiFeO₃ ), sodium niobate (NaNbO₃), bismuth titanate (Bi₄ Ti₃ O_{1 2} ), sodium bismuth titanate (Na_{0 . 5} Bi ₀ . ₅ TiO₃ ), polyvinylidene fluoride, aluminum nitride (AIN), gallium phosphate (GaPO₄ ) and gallium arsenic (GaAs).

5. The method for generating a highly reactive intermediate according to any one of claims 1 to 4, wherein the highly reactive intermediate comprises at least one selected from a radical, an anion radical and an anion.

6. A redox reaction method comprising the method for generating a highly reactive intermediate according to any one of claims 1 to 5, the redox reaction method comprising:
subjecting the electron-accepting active compound (1) to a redox reaction to produce a redox reaction product.

7. The redox reaction method according to claim 6, which comprises:
further preparing at least one compound (2) selected from an aromatic compound (2-1) optionally containing a heteroatom, a diboronic acid ester (2-2) and an aliphatic alcohol (2-3); and
applying mechanical strain to the piezoelectric material (3) in the presence of the compound (2), in addition to the electron-accepting active compound (1) and the piezoelectric material (3), and subjecting the compound (1) to one-electron reduction to generate a corresponding highly reactive intermediate, followed by a reaction of the highly reactive intermediate with the compound (2) to produce a redox reaction product.

8. The redox reaction method according to claim 7, wherein the redox reaction between the electron-accepting active compound (1) and the compound (2) is selected from:
a redox reaction between an aryl compound having a leaving group represented by the formula (1-1) as the electron-accepting active compound (1), and an aromatic compound (2-1) optionally containing a heteroatom or a diboronic acid ester (2-2) as the compound (2); and
a redox reaction between trifluoromethyl compounds represented by the formulas (I-2a) to (I-2c) as the electron-accepting active compound (1), and an aromatic compound (2-1) optionally containing a heteroatom as the compound (2).

9. The redox reaction method according to any one of claims 6 to 8, wherein the compound (2) is selected from:
an aromatic compound (2-1) optionally containing a heteroatom represented by the following general formula (11-1):
A² -H
wherein A² is selected from an optionally substituted aryl group and an optionally substituted heteroaryl group;
a diboronic acid ester (2-2) represented by the following general formula (II-2): 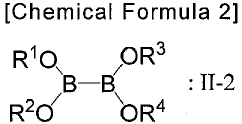 wherein R¹ to R⁴ are each independently selected from hydrogen, an optionally substituted alkyl group and an optionally substituted aryl group, R¹ and R² may be bonded to each other, and R³ and R⁴ may be bonded to each other; and
an aliphatic alcohol (2-3) represented by the following general formula (II-3): 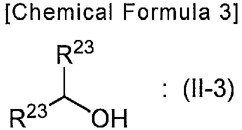 wherein R^{2 3} may be the same or different from each other, and are each independently selected from hydrogen, an optionally substituted alkyl group, an optionally substituted alkenyl group, an optionally substituted alkynyl group and an optionally substituted aryl group, and R^{2 3} and R^{2 3} may be bonded to each other.

10. The redox reaction method according to any one of claims 6 to 9, wherein the aromatic group in the aromatic compound (2-1) optionally containing a heteroatom can be selected from an optionally substituted aryl group and an optionally substituted heteroaryl group,
the optionally substituted aryl group comprises a phenyl group, a naphthyl group, an anthracenyl group, a phenanthrenyl group, a biphenyl group, a terphenyl group, a pyrenyl group, a perylenyl group, a triphenyleny group and a coronenyl group,
the optionally substituted heteroaryl group comprises a sulfur-containing heteroaryl group, an oxygen-containing heteroaryl group, a nitrogen-containing heteroaryl group, and a heteroaryl group containing two or more heteroatoms,
the diboronic acid ester (2-2) comprises a diboronic acid alkyl ester, a diboronic acid alkylene glycol ester, a diboronic acid aryl ester, a diboronic acid arylene glycol ester and tetrahydroxydiboran, and
the aliphatic alcohol (2-3) comprises a primary aliphatic alcohol and a secondary aliphatic alcohol.

11. A method for producing a redox reaction product, which comprises using the redox reaction method according to any one of claims 6 to 10.
